# EUROPEAN PATENT SPECIFICATION

(11) **EP 0 584 043 B1**
(45) Date of publication and mention of the grant of the patent: **30.10.1996**
(21) Application number: 93810562.4
(22) Date of filing: 09.08.1993
(51) Int. Cl.: C07C 2/66, C07D 521/00, A01N 47/36, C07C 311/58, C07D 239/42, C07D 251/42

(54) **Process for the preparation of substituted benzenes and benzene sulfonic acid and derivatives thereof and a process for the preparation of N'N-substituted ureas**
Verfahren zur Herstellung von substituierten Phenyl- und Phenylsulfonsäureverbindungen und ihrer Derivate und Verfahren zur Herstellung von N,N-substituierten Harnstoffen
Procédé de préparation de benzènes et d'acides benzène sulfoniques et de leurs dérivés et procédé de préparation d'urées N,N' substituées

(30) Priority: 18.08.1992 US 932135
(43) Date of publication of application: 23.02.1994
(73) Proprietor: CIBA-GEIGY AG, 4002 Basel (CH)
(72) Inventor: Baumeister, Peter, CH-4112 Flüh (CH); Seifert, Gottfried, CH-4312 Magden (CH); Steiner, Heinz, CH-4142 Münchenstein (CH)

(56) References cited:
- EP-A- 0 044 210
- EP-A- 0 120 814
- EP-A- 0 187 489
- CHEMICAL ABSTRACTS, vol. 92, 1980, Columbus, Ohio, US; abstract no. 41471x,
- CHEMICAL ABSTRACTS, vol. 85, 1976, Columbus, Ohio, US; abstract no. 123545u,
- TETRAHEDRON vol. 37, 1981, pages 31 - 36 K. KIKUKAWA ET AL

## Description

The present invention relates to an improved process for the preparation of substituted benzenes and benzenesulfonic acid and its derivatives comprising diazotisation of an ortho-amino-benzenesulfonic acid derivative followed by homogeneous palladium-catalysed coupling and heterogeneous palladium-catalysed hydrogenation, wherein the homogeneous catalyst is reduced and precipitated as metal after the coupling and used as a heterogeneous palladium catalyst for the hydrogenation step without separation. The present invention further relates to a process for the preperation N-benzenesulfonyl-N'-triazinyl-ureas.

Benzene sulfonic acid derivatives may be used as intermediates in the production of agricultural chemicals. The preparation of N-phenylsulfonyl-N'-pyrimidinyl ureas with plant growth-regulating properties from benzene sulfonic acid salts is described, for example, in EP-A-120814.

Stepwise processes are known in which an aryl-alkene coupling catalysed by Pd(0) is followed by isolation and subsequent catalytic hydrogenation. The "Heck" reaction involves the coupling of an alkene with an arylhalide, while the "Matsuda" version of the Heck reaction proceeds via more reactive adducts e.g. an aryl diazonium ion as described, for example, in Tetrahedron Vol. 37 p. 31 to 36 (1981). Stepwise procedures are described, for example, in EP-A-120814 and by M. Somei et al. in Heterocycles, vol. 26, No. 7., p. 1783 to 1784 (1987).

Nevertheless a process for the preparation of substituted benzenesulfonic acid and its derivatives in which separation of the palladium catalyst is avoided and the palladium is used in both homogeneous and heterogeneous reaction steps is not known.

Surprisingly it has now been found that a homogeneous aryl-coupling reaction with olefines catalysed by a homogeneous palladium complex can be followed by a hydrogenation step in which the palladium from homogeneous palladium complex is used for the heterogeneous hydrogenation step as Pd metal, after which the palladium may be recovered by filtration and refined by known procedures to regenerate for example the starting complex.

It is therefore an object of the invention to provide an elegant double use of the palladium catalyst and to provide a more economic process in which a soluble palladium catalyst is used in the Matsuda homogeneous step and then used in the heterogeneous hydrogenation step.

One object of the invention is a process for the preparation of compounds of the formula Ia

Ar-CHRₐ-CHR_{b}R_{c} (Ia),

wherein Rₐ, R_{b} and R_{c} are independently of each other H or a hydrogenation stable substituent and Ar means C₆-C₂₀aryl or C₃-C₂₀heteroaryl having 1 to 6 heteroatoms from the group of O, S and N, the aryl and heteroaryl being unsubstituted or substituted by hydrogenation stable residues, by
a) in a first step reacting 1 mole equivalent of a compound of the formula II

   Ar-N₂^{⊕} (IIa)

   with at least 1 mole equivalent of a compound of formula IIIa

   CHRₐ=CR_{b}R_{c} (IIIa),

   optionally in the presence of an inert solvent, and in the presence of a catalytic amount of a homogeneous palladium catalyst and a base selected from alkali metal salts, alkaline earth metal salts and a tertiary ammonium salt of a carboxylic acid to give a compound of the formula IVa

   Ar-CRₐ=CR_{b}R_{c} (IVa),

   and
b) hydrogenating in a second step the compound of the formula IVa optionally in the presence of an inert solvent and in the presence of catalytic amounts of a palladium hydrogenation catalyst, characterised in that the homogeneous palladium catalyst is reduced to insoluble palladium metal in the step a) reaction mixture, which is subsequently used as the heterogeneous hydrogenation catalyst.

A preferred variant of the process according to the invention is characterised in that the heterogeneous palladium hydrogenation catalyst is formed in situ from the homogeneous palladium catalyst in the obtained step a) reaction mixture in starting the hydrogenation by introducing hydrogen.

It is very preferred to add a palladium support material for the heterogeneous hydrogenation catalyst.

The Matsuda version of the Heck reaction works in a wide scope with compounds of the formula Ia so that substituents Rₐ, R_{b} and R_{c} may be choosen from various groups of organic residues.

Rₐ, R_{b} and R_{c} may be selected from H; C₁-C₂₀alkyl; C₁-C₂₀nitriloalkyl; C₁-C₂₀hydroxyalkyl; C₁-C₂₀halogenalkyl, halogen being preferably F, Cl or Br; C₁-C₁₂alkyl-COOR_{d}, C₁-C₁₂alkyl-CO-NRₑR_{f}, C₁-C₁₂alkyl-SO₂OR_{d} or C₁-C₁₂alkyl-SO₂-NRₑR_{f}, wherein R_{d}, R_{c} and R_{f} independently are H, C₁-C₁₂alkyl, phenyl, benzyl or cyclohexyl; C₁-C₂₀alkyl-CO; C₁-C₂₀alkoxy; C₁-C₂₀nitriloalkoxy; C₁-C₂₀halogenalkoxy, halogen being preferably F, Cl or Br; C₁-C₂₀alkylthio; C₁-C₂₀halogenalkylthio, halogen being preferably F, Cl or Br; -SO₂OR_{d}, -SO₂-NRₑR_{f}, -COOR_{d} or -CO-NRₑR_{f}, wherein R_{d}, Rₑ and R_{f} have the above meanings; halogen which is preferably F, Cl or Br; -CN; -NRₑR_{f}, wherein Rₑ and R_{f} have the above meanings; phenyl or benzyl which is unsubstituted or substituted by C₁-C₂₀alkyl; C₁-C₂₀nitriloalkyl; C₁-C₂₀hydroxyalkyl; C₁-C₂₀halogenalkyl, halogen being preferably F, Cl or Br; C₁-C₁₂alkyl-COOR_{d}, C₁-C₁₂alkyl-CO-NRₑR_{f}, C₁-C₁₂alkyl-SO₂OR_{d} or C₁-C₁₂alkyl-SO₂-NRₑR_{f}, wherein R_{d}, Rₑ and R_{f} independently are H, C₁-C₁₂alkyl, phenyl, benzyl or cyclohexyl; C₁-C₂₀alkyl-CO-; C₁-C₂₀alkoxy; C₁-C₂₀nitriloalkoxy; C₁-C₂₀halogenalkoxy, halogen being preferably F, Cl or Br; C₁-C₂₀alkylthio; C₁-C₂₀halogenalkylthio, halogen being preferably F, Cl or Br; -SO₂OR_{d}, -SO₂-NRₑR_{f}, -COOR_{d} or -CO-NRₑR_{f}, wherein R_{d}, Rₑ and R_{f} have the above meanings; halogen which is preferably F, Cl or Br; -CN; -NRₑR_{f}, wherein Rₑ and R_{f} have the above meanings. R_{d} may also represent -OM or -O(M₁)_{1/2}, where M is an alkali metal atom or a tertiary ammonium group, having from 3 to 18 carbon atoms, and M₁ is an alkaline earth metal atom. All alkyl, alkoxy and alkylthio groups contain preferably 1 to 12 carbon atoms, more preferably 1 to 8 carbon atoms and most preferably 1 to 4 carbon atoms.

Ar as aryl contains preferably 6 to 16, more preferably 6 to 12 carbon atoms and Ar may be monocyclic or condensed polycyclic aryl, whereby the polycyclic aryl may contain up to 5 and preferably up to 3 rings. Preferred Ar-groups are naphthyl and especially phenyl. The heteroaryl contains preferably 3 to 14, more preferably 3 to 10 carbon atoms, having preferably 1 to 4 and more preferably 1 to 3 heteroatoms from the group of O, S and N, whereby N is especially preferred. The heteroaryl may be monocyclic or condensed polycyclic heteroaryl, whereby the polycyclic heteroaryl may contain up to 5 and preferably up to 3 rings. Preferred heteroaryl is pyridine, triazine, pyrimydine and chinoline.

The aryl and heteroaryl may be substituted independently by the groups as mentioned above for Rₐ, R_{b} and R_{c} and also by -OH or -SH.

The reaction conditions may vary in broad range but may be preferably selected as for the more preferred object described below.

A more preferred object of the invention is a process for the preparation of compounds of the formula I

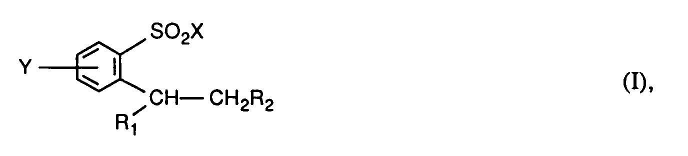

wherein X represents hydroxyl, -OM, -O(M₁)_{1/2} or NH₂, where M is an alkali metal atom or a tertiary ammonium group, having from 3 to 18 carbon atoms, and M₁ is an alkaline earth metal atom,
Y is H, Cl, F or Br,
R₁ is H, F, Cl, Br or -COOR₃,
R₂ is -COO(C₁-C₄-alkyl), -(CO)R₃ or C₁-C₂-alkyl which is unsubstituted or substituted by halogen atoms, and
R₃ is H or C₁-C₄-alkyl, by

a) in a first step reacting 1 mole equivalent of a compound of the formula II 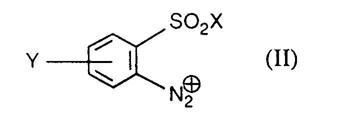 with at least 1 mole equivalent of a compound of formula III

   CHR₁=CHR₂ (III),

   optionally in the presence of an inert solvent, and in the presence of a catalytic amount of a homogeneous palladium catalyst and a base selected from alkali metal salts, alkaline earth metal salts and a tertiary ammonium salt of a carboxylic acid to give a compound of the formula IV 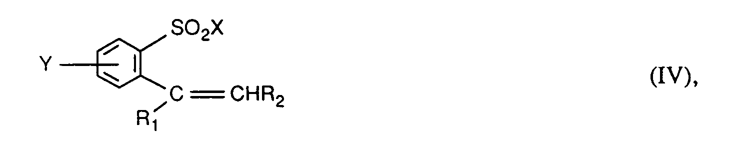 and
b) hydrogenating in a second step the compound of the formula IV optionally in the presence of an inert solvent and in the presence of catalytic amounts of a hydrogenation catalyst, characterised in that the homogeneous palladium catalyst is reduced to insoluble palladium metal in the step a) reaction mixture, which is subsequently used as the heterogeneous hydrogenation catalyst.

A preferred variant of this process is characterised in that the heterogeneous palladium hydrogenation catalyst is formed in situ from the homogeneous palladium catalyst in the obtained step a) reaction mixture in starting the hydrogenation by introducing hydrogen.

It is very preferred to add a palladium support material for the heterogeneous hydrogenation catalyst.

M in the above definition is preferably lithium, sodium or potassium. M₁ is preferably magnesium or calcium. M₁ as tertiary ammonium may be represented by the formula R₄R₅R₆NH⁺, wherein R₄, R₅ and R₆ independently are C₁-C₆-alkyl, preferably C₁-C₄-alkyl, or R₄ and R₆ together are -(CH₂)₄-, -(CH₂)₅- or -(CH₂)₂O(CH₂)₂- and R₆ is C₁-C₆-alkyl, preferably C₁-C₄-alkyl. Some examples for alkyl are methyl, ethyl, n- or i-propyl, n-, i- or t-butyl.

In the above definition alkyl denotes straight chain or branched alkyl, e.g. methyl, ethyl, n-propyl, isopropyl, and the four isomers of butyl.

The halogen substituent for R₂ as C₁-C₂-alkyl is preferably F or Cl.

R₁ is preferably H, and R₂ is preferably -CF₃, -CF₂Cl, -CFCl₂, -CCl₃, -COO(C₁-C₄-alkyl) or -(CO)CH₃. In an especially preferred embodiment, R₁ is H and R₂ is -CF₃ or -(CO)CH₃.

X is preferably OH, ONa or OK. Y is preferably H.

The starting point of the process according to the invention is an aryl diazonium cation of formula II which may be formed by methods well documented in the literature.

The diazonium compound of formula II may be formed in situ by well-known methods, or added as a salt, in which case examples of the counter anion for the compounds of formula II are PF₆⁻, BF₄⁻, OAc⁻, HSO₄⁻, SO₄²⁻, CH₃(C₆H₄)SO₃⁻ and CH₃SO₃⁻. The in situ formation may also be carried out in the presence of compounds of formula III, for example with the addition of alkylnitrites such as t-butyl nitrite as described in J. Org. Chem. Vol. 46, p. 4885 to 4888 (1981).

The palladium catalyst used in the first reaction step may be generated in situ or ex situ by reduction of a palladium(II) compound optionally in the presence of a salt such as sodium acetate and in the presence of suitable ligand-forming compounds. Suitable palladium compounds include PdCl₂, PdBr₂, Pd(NO₃)₂, H₂PdCl₄, Pd(OOCCH₃)₂, [PdCl₄]Na₂, [PdCl₄]Li₂, [PdCl₄]K₂, palladium(II)acetylacetonate, dichloro-(1,5-cyclooctadiene)palladium(II), dichlorobis-(acetonitrile)palladium(II), dichlorobis-(benzonitrile)palladium(II), π-allylpalladium(II)chloride dimer, bis-(π-methylallyl palladium(II)chloride) and π-allylpalladium(II)acetylacetonate. Suitable ligand-forming compounds are for example olefins as described by the compounds of formula III, dibenzylideneacetone (dba) unsubstituted or substituted with halogen (F, Cl and Br), C₁-C₄-alkyl or C₁-C₄-alkoxy in the benzene rings, phosphites such as those of formula P(OR₇) wherein R₇ is for example phenyl, C₁-C₆-alkyl or a partially or perfluorinated C₁-C₆-alkyl, and CO. The substituents in the benzene rings are preferably linked in the para-positions of the benzene rings. The ligand forming compounds may be used alone or in combinations of at least two compounds.

Suitable reducing agents are for example CO, H₂, formates, primary or secondary C₁-C₈-alkanols, hydrazine, amines and mixtures of CO with alkanols or water.

The catalyst may be added as Pd(dba)₂, Pd(dba)₃·solvent, Pd₂(dba)₃ or Pd₂(dba)₃·solvent, where the abbreviation "dba" stands for dibenzylidene acetone. The dba ligand may be unsubstituted or substituted in the aromatic part as described above.

The palladium catalyst may be used in an amount of about 0.01 to 5 mole %, based on the diazonium salt of formula II.

The base added in the first reaction step is used as a buffer to neutralise the acids present in the formation of the diazonium salts. The base may be used in at least equimolar amounts related to the diazonium compounds of formula II and preferably in an excess of up to 10 moles. Suitable bases are Li-, Na-, K-, NH₄-, Mg-, Ca- and NH(C₁-C₁₈-alkyl)₃-salts of carboxylic acids such as C₁-C₄-carboxylic acids or benzoic acid. Examples of suitable bases are lithium, potassium or sodium -acetate, -butyrate, -propionate and stearate, barium- and calcium acetate, calcium propionate and -stearate, lithium and sodium benzoate, and ammonium acetate; salts of acetic acid with triethylamine, tri-n-butylamine, tri-(2-ethylhexylamine), tri-n-octylamine and tri-n-dodecylamine. Especially preferred are alkaline metal acetates, which form acetic acid as a desirable component in the arylation step. Particularly preferred bases are sodium and potassium acetate in excess. The bases may also be used as salts in the catalyst generation described above.

A stoichiometric amount or small excess of alkene of formula III is preferred.

After the first reaction step, the homogeneous catalyst is reduced to form a heterogeneous catalyst. It is advantageous to use H₂ as reducing agent, since the addition of a further reactand can be avoided. It is very advantageous to add a support material for the palladium catalyst for the hydrogenation step, said support material being inert under the reaction conditions. The presence of the catalyst support or carrier can facilitate the separation of the catalyst on completion of the reaction. Examples of suitable support materials are activated carbon, carbon black, metal oxides e.g. Al₂O₃ and SiO₂, ceramics, glass and silicates e.g. synthetic and naturally occurring zeolites. Activated carbon or carbon black are preferred. The weight ratio of the catalyst support to the homogeneous palladium catalyst may be for example from 50:1 to 1:1, preferably from 20:1 to 1:1 and more preferred from 15:1 to 2:1.

The reaction temperature for the coupling step should be below the decomposition temperature of the diazonium ion, and a suitable range is between -20 and +40 °C. The hydrogenation step can be carried out between room temperature and 200 °C. To minimise side reactions it is advantageous to carry out the coupling step under elevated partial pressure of the coupling component of formula III, for example up to 10 bar, preferably between atmospheric pressure and 2 bar (1 bar = 1 x 10⁵ Pascals).

It is advantageous to carry out the hydrogenation stage of the process according to the invention at an elevated pressure, for example up to 40 bar. The hydrogen partial pressure is preferably between atmospheric pressure and 3 x 10⁶ Pascals.

Solvents for the process according to the invention may be, for example one of, or a mixture of at least one of the following: alcohols; ketones; carboxylic acids; sulfones; N,N-tetrasubstituted ureas; N-alkylated lactams or N-dialkylated acid amides; ethers; aliphatic, cycloaliphatic or aromatic hydrocarbons, which may be substituted with fluorine, chlorine, or C₁-C₄-alkyl; carboxylic acid esters and lactones; nitriles.

Some specific examples of solvents are:
alcohol: methanol, ethanol, propanol, butanol, pentanol, isopropanol, hexanol, heptanol octanol, t-butylalcohol, ethyleneglycol and diethyleneglycol.
ketone: acetone, methylethylketone, methylisobutylketone, cyclohexanone.
carboxylic acid: ethanoic acid, propanoic acid.
sulfone: dimethylsulfone, diethylsulfone, tetramethylenesulfone, sulfolan.
N,N-tetrasubstituted urea: N-methylethyl-N'-methylethylurea, N-dimethyl-N'-dipropylurea, tetramethylurea, tetraethylurea, N,N'-dimethyl-N,N'-1,3-propyleneurea, N,N'-dimethyl-N,N'-ethyleneurea.
N-alkylated lactam: N-methylpyrrolidone, N-ethylpyrrolidone.
N-dialkylated acid amide: N-dimethylformamide, N-diethylformamide, N-dimethyl-acetamide.
ether: polyethylglycolether, diethyleneglycoldimethylether, diethyleneglycoldiethylether, tetrahydrofuran, dioxan, methyl-t-butylether, diethyleneglycolmonomethylether and ethyleneglycolmonomethylether.
aliphatic hydrocarbon: methylene chloride, pentane, hexane.
cycloaliphatic hydrocarbon: cyclohexane, decahydronaphthalene.
aromatic hydrocarbon: xylene, tetrahydronaphthalene, dichlorobenzene.
carboxylic acid ester: benzoic-methylester, ethylacetate, γ-butyrolactone, n-butylacetate. nitrile: acetonitrile, benzonitrile, phenylacetonitrile.

It may be advantageous to use an ether/water, an ether/alcohol or an alcohol/water mixture as solvent for the diazotisation. The arylation step is preferably carried out under water-free reaction conditions. Water present in the diazotisation is preferably removed by the addition of carboxylic acid anhydrides such as acetic anhydride or by other well-known methods.

A further object of the invention is the reaction procedure in an alcohol as solvent, for example pentanol or isopropanol, which is surprising in view of the observation made in Tetrahedron Vol. 37, p. 31 (1981) that the use of alcoholic solvent caused reduction of diazonium salts.

Preferred solvents are butanol, pentanol, isopropanol, acetonitrile, ethanoic acid and dioxan or mixtures of these solvents.

A preferred embodiment of the process according to the invention is that the reaction is carried out as a one-pot reaction.

The process according to the invention has the following advantages:
i) The catalytic material is used in two consecutive and different reaction steps. The homogeneous catalyst for the Matsuda reaction is converted in situ into the necessary heterogeneous hydrogenation catalyst for the next step.
ii) The catalyst is recovered by filtration at the end of the hydrogenation step.
iii) More efficient use is made of the catalytic material.
iv) The palladium catalyst may be recycled from the reaction medium with negligible loss.
v) Mild conditions are used.
vi) Purer product is obtained in a higher yield.
vii) Elegant double use of expensive palladium is achieved.
viii) The process can be carried out in alcoholic solvents.
ix) Isolation of intermidiate is avoided.
x) Economic production of herbicides (sulfonyl-ureas) on an industrial scale.

It is desirable to recycle the catalyst following hydrogenation. This can be achieved by known methods.

A further object of the invention is a process for the manufacture of compounds of the formula V 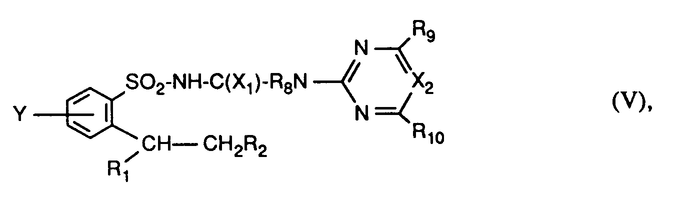 wherein
X₁ is S or O, X₂ is N or CH,
Y is H, Cl, F or Br,
R₁ is H, F, Cl, Br or -COOR₃,
R₂ is -COO(C₁-C₄-alkyl), -(CO)R₃ or C₁-C₂-alkyl which is unsubstituted or substituted by halogen atoms, and
R₃ is H or C₁-C₄-alkyl,
R₈ is H, C₁-C₃alkyl or C₁-C₃alkoxy
R₉ is C₁-C₃alkyl, C₁-C₃haloalkyl, C₁-C₃alkoxy or C₁-C₃haloalkoxy, and
R₁₀ is H, halogen, NH₂, NH(C₁-C₃alkyl), NH(C₁-C₃alkyl)₂, C₁-C₃alkyl, C₁-C₃haloalkyl,
C₁-C₃alkoxy or C₁-C₃haloalkoxy, by

a) reacting in a first step 1 mole equivalent of a compound of the formula IIb 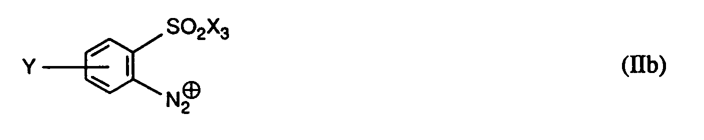 wherein X₃ represents hydroxyl, -OM or -O(M₁)_{1/2}, where M is an alkali metal atom or a tertiary ammonium group, having from 3 to 18 carbon atoms, and M₁ is an alkaline earth metal atom, with at least 1 mole equivalent of a compound of formula IIIb

   CHR₁=CHR₂ (IIIb),

   optionally in the presence of an inert solvent, and in the presence of a catalytic amount of a homogeneous palladium catalyst and a base selected from alkali metal salts, alkaline earth metal salts and a tertiary ammonium salt of a carboxylic acid to give a compound of the formula IVb 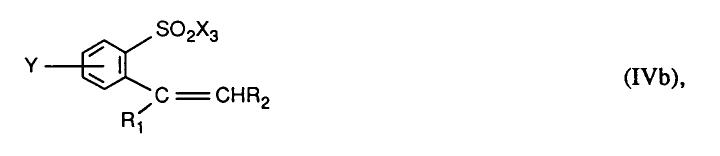 and
b) hydrogenating in a second step the compound of the formula IVb optionally in the presence of an inert solvent and in the presence of catalytic amounts of a hydrogenation catalyst, to form a compound of the formula Ib 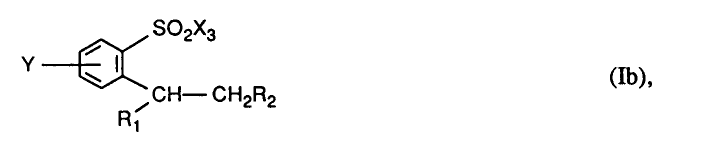
c) reacting in a third step the compound of formula Ib with at least 1 mole of a halogenating agent to form the sulfochloride, which is then reacted with NH₃ to give the sulfonamide of the formula Ic 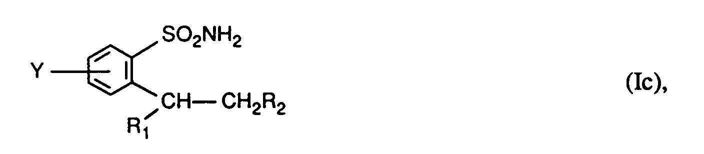
d) reacting the compound of the formula Ic with COCl₂ or CSCl₂ to obtain a compound of the formula VI 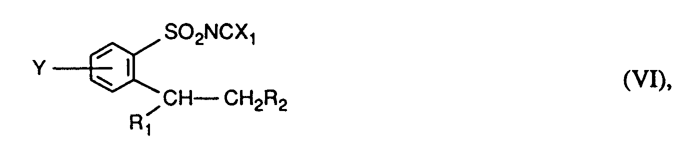 and
e) reacting the compound of the formula VI with a compound of the formula VII 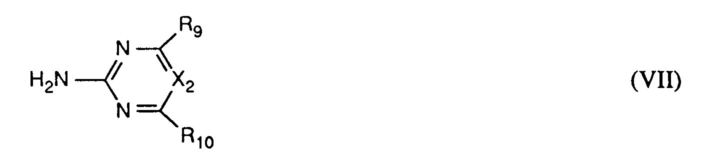 to form the compound of the formula V,
   characterised in that the homogeneous palladium catalyst is reduced to insoluble palladium metal in the step a) reaction mixture, which is subsequently used as the heterogeneous hydrogenation catalyst.

A preferred variant of this process is characterised in that the heterogeneous palladium hydrogenation catalyst in the step b) reaction is formed in situ from the homogeneous palladium catalyst in the obtained step a) reaction mixture in starting the hydrogenation by introducing hydrogen.

It is very preferred to add prior to the start of the hydrogenation a solid palladium support material for the heterogeneous hydrogenation catalyst.

The preferred embodiments for the production of compounds of the formula I applies also in the above steps a) and b) reactions. X₃ preferably represents hydroxyl or a group -OM, wherein M is is an alkali metal, preferably K or Na.

X₁ is preferably O. X₂ is preferably N. R₈ is preferably H. R₉ is preferably C₁-C₃alkyl, especially methyl or ethyl. R₁₀ is preferably C₁-C₃alkyl, especially methyl or ethyl, or C₁-C₃alkoxy, especially methoxy or ethoxy.

The process is especially used for the production of N-(4-methoxy-6-methyl-1,3,5-triazine-2-yl)-N'-[2-(3,3,3-trifluoroprop-1-yl)-benzenesulfonyl]-urea.

Reaction steps c), d) and e) are well known and described for example in US-A-4 780 125. More preferred embodiments of these reaction steps are described below.

In the step c) reaction the preferred halogenating agent is COCl₂ which may be used in excess, for example 2 to 3 mole excess. The reaction may be catalyzed by the addition of N-dialkyl carboxylic acid amides like dimethylformamide, or by lactames like N-methylpyrrolidone. Catalytic amounts are for example 0.001 to 10 mole percent related to the amount of compound Ib. The reaction can be carried out under normal pressure or elevated pressure of up to 10 bar, preferably up to 5 bar. The temperature may be from 20 to 150 °C, preferably 60 to 120 °C. Solvents may be used as those mentioned before. Preferred are halogenated hydrocarbons, especially chlorobenzene.

The sulfochloride is preferably treated without isolation in the obtained reaction mixture with acqueous NH₃ in a concentration of preferably 20 to 40 % at temperatures of preferably 20 to 100 °C, more preferably 40 to 80 °C. After cooling of the reaction mixture the compound of formula Ic precipitates and may be filtered off.

The step d) reaction is preferably carried out with an excess of phosgene or thiophosgene, for example 2 to 5 moles and preferably 2 to 3 moles. The reaction temperature is preferably 50 to 180 °C and more preferably 70 to 150 °C. The reaction is preferably catalyzed by the addition of aliphatic or cycloaliphatic isocyanates having 1 to 10 carbon atoms like cyclohexylisocyanate. Catalytic amounts are for example 0.001 to 10 mole percent related to the amount of compound Ic. The reaction can be carried out under normal pressure or elevated pressure of up to 10 bar, preferably up to 5 bar. Solvents may be used as those mentioned before. Preferred are halogenated hydrocarbons, especially chlorobenzene.

The step e) reaction is preferably carried out in the presence of a solvent as those previously mentioned, especially halogenated hydrocarbons as chlorobenzene. A preferred temperature range is from 20 to 180 °C, especially 50 to 150 °C. The reaction is in general carried out under normal pressure or an elevated pressure of up to 1 bar. Equivalent molar ratios of the compounds of the formulae VI and VII are preferred. In a preferred embodiment the obtained reaction solution with the isocyanate of formula VI is added to the solution or suspension of the compound of the formula VII. After cooling of the reaction mixture the compound of the formula V may be filtered off and may be purified by washing the filter cake with a mineral acid like hydrochloric acid and then with an alcanol like methanol. The product is obtained in high yields (90 % or more) and purity (content at least 95 % and up to more than 99 %). The inventive process is economic, ecologic, technically feasible and save even on an industrial scale.

The following examples illustrate the invention.

### Example 1 Preparation of sodium trifluoropropyl-benzenesulfonate (in isopropanol)

### a) Preparation of diazosulfonate

173.3 g aniline-2-sulfonic acid (1 mol) are stirred into 750 g isopropanol (IPA) together with 75 g water at 15 to 20 °C in a 1.5 dm³ double-sleeve vessel. 89 g isopropylnitrite (IPN*) (1 mol) are added dropwise to the reaction mixture over 60 minutes while stirring is continued at between 15 and 20 °C. Any unreacted IPN is consumed by the addition of dilute aniline-2-sulfonic acid. The diazo suspension obtained is cooled and stored at between 0 and 5 °C.
* IPN may be made by the reaction of isopropanol with sodium nitrite and HCl at 0 °C.

### b) Preparation of sodium trifluoropropenyl-benzenesulfonate

The diazo suspension from 1a) is transferred to a pressure vessel equipped with a pressure regulator. 123 g dry sodium acetate (1.5 mol) are added and the mixture stirred for 1 hour. 3 g Pd(dba)₂ (0.005 mol) are added, the mixture stirred for 5 minutes and the reaction vessel closed. At 1 bar and with the temperature between 5 and 10 °C 106 g 3,3,3-trifluoropropene (1.1 mol) are introduced over a 4 hour period. After the first hour the temperature is increased to 27 to 28 °C and kept there until no more nitrogen is evolved. Approximately 25 dm³ gas are evolved. The reaction mixture is transferred into a double-sleeved reaction vessel, 500 ml water added and the isopropanol is distilled off as an azeotrope isopropanol/water at atmospheric pressure. The aqueous solution containing 255 g sodium trifluoropropenyl-benzenesulfonate is cooled to room temperature.

### c) Preparation of sodium trifluoropropyl-benzenesulfonate

The reaction mixture from 1b) is transferred into a hydrogenation autoclave and 20 g activated carbon are added. The hydrogenation is carried out at 1 bar and 30 to 40 °C for 6 to 8 hours. The catalyst is filtered off and washed with 100 ml water. The aqueous filtrate contains 256 g of the title compound, determined by high pressure liquid chromatography. The aqueous solution of sodium trifluoropropyl-benzenesulfonate (1020 g) can be converted to the corresponding sulfonamide (1d) via the acid chloride.

### d) Characterisation of the title compound

The title sodium salt may be characterised by conversion to the corresponding sulfonamide via the respective acid chloride.

1020 g 27 % aqueous solution of sodium trifluoropropyl benzene sulfonate (1 mol) are acidified with 75 g 32 % HCl to pH 1. 400 g water are evaporated off at 55 to 62 °C under 150 mbar vacuum. 1385 g chlorobenzene are added and a further 235 g water removed under 280 mbar vacuum. 15.4 ml dimethylformamide are added and the reaction mixture heated to between 100 and 105 °C. The temperature remains at this level and 281 g phosgene are admitted over a 10-hour period. After 30 min stirring, the vessel is evacuated to 400 mbar and 215 ml chlorobenzene distilled off at between 90 and 105 °C. The suspension is filtered at room temperature and washed with chlorobenzene. The clear brown filtrate containing the corresponding sulfochloride is warmed to between 55 and 60 °C and 155 g 30 % ammonia added dropwise over a 30 min period. After stirring for a further 15 min, 4.5 g activated charcoal are added. About 106 g water are evaporated off and the dry suspension diluted with 410 ml chlorobenzene. The suspension (NH₄Cl) is filtered over a prewarmed suction filter treated with hyflo and the filtercake washed with 165 ml hot chlorobenzene. After crystallisation the suspension is cooled slowly to between 0 and 5 °C. The suspension is stirred for 30 min and filtered. The filtercake is washed with cold chlorobenzene and dried in a vacuum chamber at 70 °C. 229 g trifluoropropyl benzene sulfonamide are obtained.

### Example 2 Preparation of sodium trifluoropropyl-benzenesulfonate (in ethanoic acid)

### a) Preparation of diazosulfonate

244.6 g 88.5 % aniline-2-sulfonic acid (1.25 mol) are stirred into 900 ml dry ethanoic acid at room temperature. 85.8 g 96 % sulfuric acid are run into the reaction mixture over 30 minutes while stirring is continued. The suspension is cooled to between 18 and 20 °C. 215.6 g 40 % aqueous sodium nitrite solution (1.25 mol) are added dropwise to the reaction mixture at between 18 and 20 °C which is stirred for a further 30 minutes. 3 ml 16.7 % aqueous aniline-2-sulfonic acid are stirred in to consume any excess nitrite. Over a period of 3 hours at between 20 and 24 °C, 497.5 g ethanoic anhydride (4.87 mol) are added, and after stirring for a further 1 hour, the resulting yellow suspension is cooled to between 12 and 15 °C.

### b) Preparation of sodium trifluoropropenyl-benzenesulfonate

The diazo suspension from 2a) is transferred to a 2.5 dm³ vessel. At between 15 and 17 °C 220 g sodium acetate (2.68 mol) are added and the mixture is stirred for 1 hour. The temperature rises to 20 to 24 °C and stirring is continued for 45 minutes. With the temperature at 24 °C, 3.6 g Pd(dba)₂ are added and the mixture stirred for 5 minutes. 130 g 3,3,3-trifluoropropene (1.35 mol) are introduced over a 4 hour period. A mildly exothermic reaction follows and the temperature remains at between 25 and 28 °C for a further 30 minutes until no more nitrogen is evolved. Approximately 34 dm³ gas are evolved. The ethanoic acid is distilled off under vacuum (200 mbar) at a temperature of 70 to 90 °C. When the distillation residue weight has fallen to between 850 and 900 g, 550 ml water are added and the mixture stirred at between 60 and 65 °C.

### c) Preparation of sodium trifluoropropyl-benzenesulfonate

The reaction mixture from 2b) is transferred into a hydrogenation autoclave and 35 g activated carbon are added. The hydrogenation is carried out at a pressure of 1 bar and a temperature of between 30 and 40 °C for 6 to 8 hours. The palladium-containing catalyst is separated by filtration and washed with 120 ml water; the aqueous filtrate contains 314 g of the title compound, determined by HPLC, and less than 2 ppm Pd. The aqueous solution of sodium trifluoropropyl-benzenesulfonate can be converted directly to the corresponding sulfonamide as described in 1d) or isolated as follows:
The aqueous solution of sodium trifluoropropyl-benzenesulfonate is concentrated to 800 g. At 65 to 70 °C approximately 330 g 30 % NaOH are added until a pH of 9 is reached, when the product precipitates. After cooling to room temperature the suspension is filtered and washed with 400 ml 25 % NaCl brine in 4 portions. The wet cake is dried in a vacuum oven at 80 °C. 420 g sodium trifluoropropenyl-benzenesulfonate are obtained (assay 70 % determined by LC analysis).

### Example 3: Preperation of N-(4-methoxy-6-methyl-1,3,5-tiazine-2-yl)-N'-[2-(3,3,3-trifluoroprop-1-yl)-benzenesulfonyl]-urea in a pilot plant.

The following reactions are carried out in enamelled 630 l vessels.

### a) Preperation of sodium-[2-(3,3,3-trifluoro-1-propenyl)]-benzene-sulfonate.

Aniline-2-sulfonic acid is diazotised with pentylnitrite (molar ratio 1:1.05) at 15 to 20 °C in pentanol containing up to 10% water as solvent. Excess pentylnitrite is destroyed with sulfamic acid and the water is converted to acetic acid by adding acetic acid anhydride. Sodium acetate (molar ratio aniline-2-sulfonic acid to sodium acetate 1:2) is added and stirring is continued for 90 minutes at 20 to 30 °C.
In a seperate stainless steel vessel, dibenzylideneacetone (molar ratio diazonium salt to dibenzylideneacetone 1:0.04) and sodium acetate (molar ratio diazonium salt to sodium acetate 1:0.1) are mixed in pentanole and a solution of palladium dichloride (molar ratio diazonium salt to palladium dichloride 1:0.01) is added at 60 °C. After cooling to 30 °C the mixture is added to the suspended diazonium salt. 3,3,3-trifluoropropene (molar ratio diazonium salt to 3,3,3-trifluoropropene 1:1.01) is introduced during 5 hours and stirring is continued until no diazonium salt can be detected. The suspension is then ready for hydrogenation.

### b) Preperation of sodium-[2-(3,3,3-trifluoro-prop-1-yl)]-benzene-sulfonate.

Charcoal (weight ratio sodium-[2-(3,3,3-trifluoro-1-propenyl)]-benzene-sulfonate to charcoal 10:1) is added to the above suspension and hydrogen is introduced during 6 hours at 35 to 40 °C and a pressure of 1 bar. Suspended material is filtered off and the pentanol solution is washed with water/sodium hydroxide to remove sodium acetate and byproducts. Pentanole is partially distilled off, water is added and the remaining pentanole is removed by azeotropic destillation. The resulting solution of the product in water is used in the next step.

### c) Preperation of 2-(3,3,3-trifluoro-prop-1-yl)]-benzene-sulfonamide.

Water is distilled off from the above solution. Chlorobenzene is added and the remaining water is removed by azeotropic destillation. Phosgene (molar ratio of sodium-[2-(3,3,3-trifluoro-prop-1-yl)]-benzene-sulfonate to phosgene 1:2.5) is introduced at 85 to 105 °C during 5 hours in the presence of catalytic amounts of dimethylformamide (molar ratio phosgene to dimethylformamide 1:0.1). The solution is then treated with an excess of aqueous NH₃ (content 30%, molar ratio 2-(3,3,3-trifluoro-prop-1-yl)-benzene-sulfochloride to NH₃ 1:4) at 60 °C during 1 hour and the reaction mixture is stirred for further 2 hours. After cooling the precipitated is filtered off and used in the next step.

### d) Preperation of N-(4-methoxy-6-methyl-1,3,5-tiazine-2-yl)-N'-[2-(3,3,3-trifluoroprop-1-yl)-benzenesulfonyl]-urea.

The product of the previous step is suspended in hot chlorobenzene. In the presence of catalytic amounts of cyclohexylisocyanate (molor ratio of product to cyclohexylisocyanate 1:0.01) phosgene (molor ratio of product to phosgene 1:3) is introduced at 100 to 120 °C during 5 hours. The chlorobenzene is destilled off until a concentration of 25 % isocyanate is reached. This solution is added during 1 hour to a suspension of 2-amino-4-methyl-6-methoxy-triazine in chlorobenzene at 90 °C. The suspension is stirred for further 90 minutes and then cooled. The product is filtered off and vacuum dried. 170 kg of pure product are prepared at the pilot plant.

## Claims

1. A process for the preparation of compounds of the formula Ia
Ar-CHRₐ-CHR_{b}R_{c} (Ia),
wherein Rₐ, R_{b} and R_{c} are independently of each other H or a hydrogenation stable substituent and Ar means C₆-C₂₀aryl or C₃-C₂₀heteroaryl having 1 to 6 heteroatoms from the group of O, S and N, the aryl and heteroaryl being unsubstituted or substituted by hydrogenation stable residues, by
a) in a first step reacting 1 mole equivalent of a compound of the formula II
Ar-N₂^{⊕} (IIa)
with at least 1 mole equivalent of a compound of formula IIIa
CHRₐ=CR_{b}R_{c} (IIIa),
optionally in the presence of an inert solvent, and in the presence of a catalytic amount of a homogeneous palladium catalyst and a base selected from alkali metal salts, alkaline earth metal salts and a tertiary ammonium salt of a carboxylic acid to give a compound of the formula IVa
Ar-CRₐ=CR_{b}R_{c} (IVa),
and
b) hydrogenating in a second step the compound of the formula IVa optionally in the presence of an inert solvent and in the presence of catalytic amounts of a palladium hydrogenation catalyst, characterised in that that the homogeneous palladium catalyst is reduced to insoluble palladium metal after the step a) reaction, which is subsequently used as the heterogeneous hydrogenation catalyst.

2. A process according to claim 1, wherein the heterogeneous palladium hydrogenation catalyst is formed in situ from the homogeneous palladium catalyst in the obtained step a) reaction mixture in starting the hydrogenation by introducing hydrogen.

3. A process according to claim 1, wherein a palladium support material for the heterogeneous hydrogenation catalyst is added prior to the introduction of hydrogene.

4. A process according to claim 1, wherein Rₐ, R_{b} and R_{c} are selected from H; C₁-C₂₀alkyl; C₁-C₂₀nitriloalkyl; C₁-C₂₀hydroxyalkyl; C₁-C₂₀halogenalkyl, halogen being preferably F, Cl or Br; C₁-C₁₂alkyl-COOR_{d}, C₁-C₁₂alkyl-CO-NRₑR_{f}, C₁-C₁₂alkyl-SO₂OR_{d} or C₁-C₁₂alkyl-SO₂-NRₑR_{f}, wherein R_{d}, Rₑ and R_{f} independently are H, C₁-C₁₂alkyl, phenyl, benzyl or cyclohexyl; C₁-C₂₀alkyl-CO; C₁-C₂₀alkoxy; C₁-C₂₀nitriloalkoxy; C₁-C₂₀halogenalkoxy, halogen being preferably F, Cl or Br; C₁-C₂₀alkylthio; C₁-C₂₀halogenalkylthio, halogen being preferably F, Cl or Br; -SO₂OR_{d}, -SO₂-NRₑ R_{f}, -COOR_{d} or -CO-NRₑR_{f}, wherein R_{d}, Rₑ and R_{f} have the above meanings; halogen which is preferably F, Cl or Br; -CN; -NRₑR_{f}, wherein Rₑ and R_{f} have the above meanings; phenyl or benzyl which is unsubstituted or substituted by C₁-C₂₀alkyl; C₁-C₂₀nitriloalkyl; C₁-C₂₀hydroxyalkyl; C₁-C₂₀halogenalkyl, halogen being preferably F, Cl or Br; C₁-C₁₂alkyl-COOR_{d}, C₁-C₁₂alkyl-CO-NRₑR_{f}, C₁-C₁₂alkyl-SO₂OR_{d} or C₁-C₁₂alkyl-SO₂-NRₑR_{f}, wherein R_{d}, Rₑ and R_{f} independently are H, C₁-C₁₂alkyl, phenyl, benzyl or cyclohexyl; C₁-C₂₀alkyl-CO-; C₁-C₂₀alkoxy; C₁-C₂₀nitriloalkoxy; C₁-C₂₀halogenalkoxy, halogen being preferably F, Cl or Br; C₁-C₂₀alkylthio; C₁-C₂₀halogenalkylthio, halogen being preferably F, Cl or Br; -SO₂OR_{d}, -SO₂-NRₑR_{f}, -COOR_{d} or -CO-NRₑR_{f}, wherein R_{d}, Rₑ and R_{f} have the above meanings; halogen which is preferably F, Cl or Br; -CN; -NRₑR_{f}, wherein Rₑ and R_{f} have the above meanings, whereby R_{d} can also represent -OM or -O(M₁)_{1/2}, where M is an alkali metal atom or a tertiary ammonium group, having from 3 to 18 carbon atoms, and M₁ is an alkaline earth metal atom.

5. A process according to claim 4, wherein the alkyl, alkoxy and alkylthio groups contain 1 to 12 carbon atoms.

6. A process according to claim 1, wherein the aryl Ar contains 6 to 16 carbon atoms and Ar is monocyclic or condensed polycyclic aryl, whereby the polycyclic aryl contains up to 3 rings.

7. A process according to claim 6, wherein the Ar-groups are naphthyl or phenyl.

8. A process according to claim 1, wherein the heteroaryl contains 3 to 14 carbon atoms, having 1 to 4 heteroatoms from the group of O, S and N.

9. A process according to claim 1, wherein the heteroaryl is monocyclic or condensed polycyclic heteroaryl, whereby the polycyclic heteroaryl contains up to 3 rings.

10. A process according to claim 1 for the preparation of compounds of the formula I 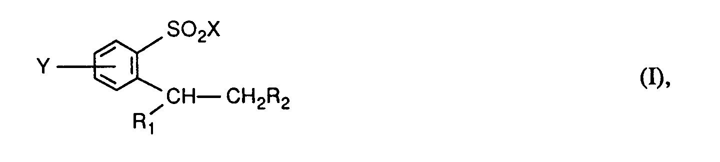 wherein
X represents hydroxyl, -OM, -O(M₁)_{1/2} or NH₂, where M is an alkali metal atom or a tertiary ammonium group, having from 3 to 18 carbon atoms, and M₁ is an alkaline earth metal atom,
Y is H, Cl, F or Br,
R₁ is H, F, Cl, Br or -COOR₃,
R₂ is -COO(C₁-C₄-alkyl), -(CO)R₃ or C₁-C₂-alkyl which is unsubstituted or substituted by halogen atoms, and
R₃ is H or C₁-C₄-alkyl, by
a) in a first step reacting 1 mole equivalent of a compound of the formula II 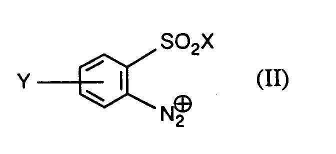 with at least 1 mole equivalent of a compound of formula III
CHR₁=CHR₂ (III),
optionally in the presence of an inert solvent, and in the presence of a catalytic amount of a homogeneous palladium catalyst and a base selected from alkali metal salts, alkaline earth metal salts and a tertiary ammonium salt of a carboxylic acid to give a compound of the formula IV 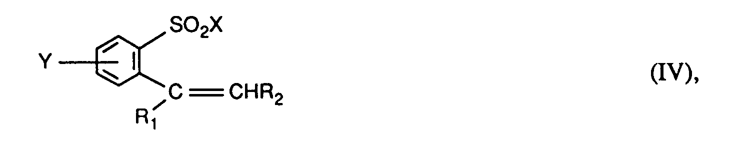 and
b) hydrogenating in a second step the compound of the formula IV optionally in the presence of an inert solvent and in the presence of catalytic amounts of a hydrogenation catalyst, characterised in that the homogeneous palladium catalyst is reduced to insoluble palladium metal in the step a) reaction mixture, which is subsequently used as the heterogeneous hydrogenation catalyst.

11. A process according to claim 10, wherein the heterogeneous palladium hydrogenation catalyst is formed in situ from the homogeneous palladium catalyst in the obtained step a) reaction mixture in starting the hydrogenation by introducing hydrogen.

12. A process according to claim 10, wherein M is lithium, sodium or potassium.

13. A process according to claim 10, wherein M₁ is magnesium or calcium.

14. A process according to claim 10, wherein R₁ is H.

15. A process according to claim 10, wherein R₂ is -CF₃, -CF₂Cl, -CFCl₂,-CCl₃, -COO(C₁-C₄-alkyl) or -(CO)CH₃.

16. A process according to claim 1, wherein R₁ is H and R₂ is -CF₃ or -(CO)CH₃.

17. A process according to claim 1, wherein X is OH, ONa or OK.

18. A process according to claim 1, wherein Y is H.

19. A process according to claim 1, wherein the catalyst is generated in situ or ex situ by reduction of a palladium(II) compound and in the presence of suitable ligand-forming compounds.

20. A process according to claim 19, wherein the palladium compounds include PdCl₂, PdBr₂, Pd(NO₃)₂, H₂PdCl₄, Pd(OOCCH₃)₂, [PdCl₄]Na₂, [PdCl₄]Li₂, [PdCl₄]K₂, palladium(II)acetylacetonate, dichloro-(1,5-cyclooctadiene)palladium(II), dichlorobis-(acetonitrile)palladium(II), dichlorobis-(benzonitrile)palladium(II), π-allylpalladium(II)chloride dimer, bis-(π-methylallyl palladium(II)chloride) and π-allylpalladium(II)acetylacetonate.

21. A process according to claim 19, wherein the ligand-forming compounds are selected from the group of olefins as described by the compounds of formula III, dibenzylidene-acetone (dba) unsubstituted or substituted with halogen, C₁-C₄-alkyl or C₁-C₄-alkoxy in the benzene rings, phosphites of formula P(OR₇) wherein R₇ is for example phenyl, C₁-C₆-alkyl or a partially or perfluorinated C₁-C₆-alkyl, and CO.

22. A process according to claim 10, wherein the reducing agents used are CO, H₂, formates, primary or secondary C₁-C₈-alkanols, hydrazine, amines and mixtures of CO with alkanols or water.

23. A process according to claim 10, wherein the catalyst added is Pd(dba)₂, Pd(dba)₃·solvent, Pd₂(dba)₃ or Pd₂(dba)₃·solvent, where the abbreviation "dba" stands for dibenzylidene acetone, which is unsubstituted or substituted in the aromatic part as defined in claim 21.

24. A process according to claim 10, wherein the palladium catalyst is used in an amount of 0.01 to 5 mole %, based on the diazonium salt of formula II according to claim 10.

25. A process according to claim 10, wherein a base is present which is selected from Li-, Na-, K-, NH₄-, Mg-, Ca- and NH(C₁-C₁₈-alkyl)₃-salts of carboxylic acids.

26. A process according to claim 25, wherein the carboxylic acid is a C₁-C₄-carboxylic acid or benzoic acid.

27. A process according to claim 25, wherein sodium or potassium acetate is present as the base.

28. A process according to claim 10, wherein a palladium support material for the heterogeneous hydrogenation catalyst is added prior to the introduction of hydrogene.

29. A process according to claim 28, wherein activated carbon, carbon black, Al₂O₃, SiO₂, a ceramic, glass, or synthetic or naturally occurring zeolite is added as catalyst support material.

30. A process according to claim 10, wherein a small excess of alkene of formula III is used.

31. A process according to claim 10, wherein the reaction temperature for the coupling step is between -20 and +40 °C.

32. A process according to claim 10, wherein the reaction temperature for the hydrogenation step is between room temperature and 200 °C.

33. A process according to claim 10, wherein the coupling step is carried out at a partial pressure of the coupling component of formula III of between atmospheric pressure and 10⁶ Pascals.

34. A process according to claim 10, wherein the hydrogenation stage is carried out at a hydrogen partial pressure of between atmospheric pressure and 3 x 10⁶ Pascals.

35. A process according to claim 10, wherein the reaction is carried out in an alcohol as solvent.

36. A process according to claim 35, wherein the solvent is pentanol or isopropanol.

37. A process according to claim 10, wherein the reaction is carried out in butanol, pentanol, isopropanol, acetonitrile, ethanoic acid or dioxan as solvent.

38. A process according to claim 10, wherein the reaction is carried out as a one-pot reaction.

39. A process according to claim 10, wherein the homogeneous palladium catalyst is reduced with hydrogen.

40. A process according to claim 10 for the preperation of sodium-2-(3,3,3-trifluoroprop-1-yl)-benzene-sufonate.

41. A process for the manufacture of compounds of the formula V 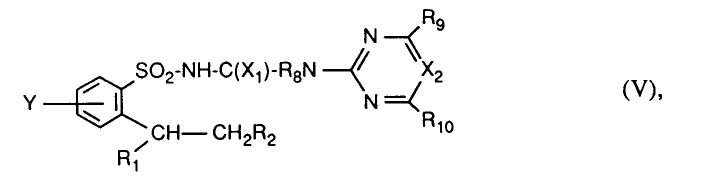 wherein
X₁ is S or O, X₂ is N or CH,
Y is H, Cl, F or Br,
R₁ is H, F, Cl, Br or -COOR₃,
R₂ is -COO(C₁-C₄-alkyl), -(CO)R₃ or C₁-C₂-alkyl which is unsubstituted or substituted by halogen atoms, and
R₃ is H or C₁-C₄-alkyl,
R₈ is H, C₁-C₃alkyl or C₁-C₃alkoxy
R₉ is C₁-C₃alkyl, C₁-C₃haloalkyl, C₁-C₃alkoxy or C₁-C₃haloalkoxy, and
R₁₀ is H, halogen, NH₂, NH(C₁-C₃alkyl), NH(C₁-C₃alkyl)₂, C₁-C₃alkyl,
C₁-C₃haloalkyl, C₁-C₃alkoxy or C₁-C₃haloalkoxy, by
a) reacting in a first step 1 mole equivalent of a compound of the formula IIb 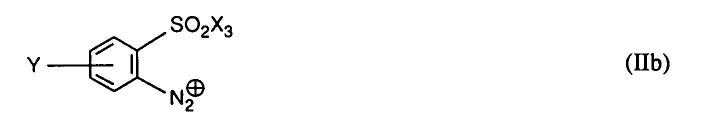 wherein X₃ represents hydroxyl, -OM or -O(M₁)_{1/2}, where M is an alkali metal atom or a tertiary ammonium group, having from 3 to 18 carbon atoms, and M₁ is an alkaline earth metal atom, with at least 1 mole equivalent of a compound of formula IIIb
CHR₁=CHR₂ (IIIb),
optionally in the presence of an inert solvent, and in the presence of a catalytic amount of a homogeneous palladium catalyst and a base selected from alkali metal salts, alkaline earth metal salts and a tertiary ammonium salt of a carboxylic acid to give a compound of the formula IVb 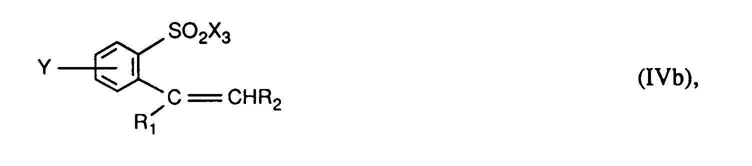 and
b) hydrogenating in a second step the compound of the formula IVb optionally in the presence of an inert solvent and in the presence of catalytic amounts of a hydrogenation catalyst, to form a compound of the formula Ib 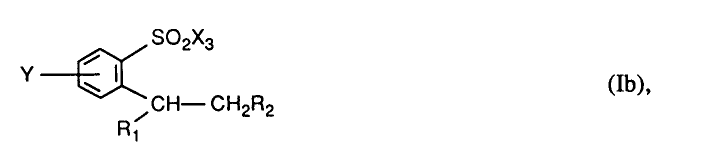
c) reacting in a third step the compound of formula Ib with at least 1 mole of a halogenating agent to form the sulfochloride, which is then reacted with NH₃ to give the sulfonamide of the formula Ic 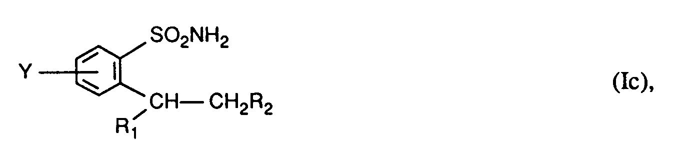
d) reacting the compound of the formula Ic with COCl₂ or CSCl₂ to obtain a compound of the formula VI 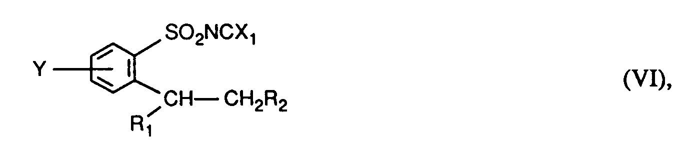 and
e) reacting the compound of the formula VI with a compound of the formula VII 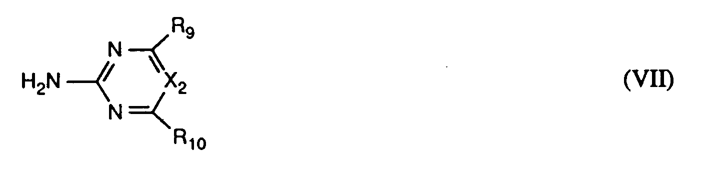 to form the compound of the formula V,
characterised in that the homogeneous palladium catalyst is reduced to insoluble palladium metal in the step a) reaction mixture, which is subsequently used as the heterogeneous hydrogenation catalyst.

42. A process according to claim 41, characterised in that the heterogeneous palladium hydrogenation catalyst in the step b) reaction is formed in situ from the homogeneous palladium catalyst in the obtained step a) reaction mixture in starting the hydrogenation by introducing hydrogen.

43. A process according to claim 41, wherein a solid palladium support material for the heterogeneous hydrogenation catalyst is added prior to the start of the hydrogenation.

44. A process according to claim 41, wherein X₃ represents hydroxyl or a group -OM, wherein M is is an alkali metal.

45. A process according to claim 41, wherein X₃ represents the group -ONa.

46. A process according to claim 41, wherein X₁ is O.

47. A process according to claim 41, wherein X₂ is N.

48. A process according to claim 41, wherein R₈ is H.

49. A process according to claim 41, wherein R₉ is C₁-C₃alkyl.

50. A process according to claim 49, wherein R₉ is methyl or ethyl.

51. A process according to claim 41, wherein R₁₀ is C₁-C₃alkyl or C₁-C₃alkoxy.

52. A process according to claim 51, wherein R₁₀ is methyl, ethyl, methoxy or ethoxy.

53. A process according to claim 41 for the production of N-(4-methoxy-6-methyl-1,3,5-triazine-2-yl)-N'-[2-(3,3,3-trifluoroprop-1-yl)-benzenesulfonyl]-urea.

54. A process according to claim 41, wherein in the step c) reaction the halogenating agent is COCl₂.

55. A process according to claim 54, wherein the reaction is catalyzed by the addition of dimethylformamide.

56. A process according to claim 55, wherein 0.001 to 10 mole percent of dimethylformamide are used related to the amount of compound Ib.

57. A process according to claim 54, wherein the reaction temperature is from 20 to 150 °C.

58. A process according to claim 54, wherein chlorobenzene is used as solvent.

59. A process according to claim 54, wherein the sulfochloride is treated without isolation in the obtained reaction mixture with acqueous NH₃ to form the sulfonamide of formula Ic.

60. A process according to claim 59, wherein the reaction temperature is from 20 to 100 °C.

61. A process according to claim 41, wherein the step d) reaction is carried out with an excess of phosgene or thiophosgene.

62. A process according to claim 61, wherein the reaction temperature is from 50 to 180 °C.

63. A process according to claim 61, wherein the reaction is catalyzed by the addition of aliphatic or cycloaliphatic isocyanates having 1 to 10 carbon atoms.

64. A process according to claim 63, wherein cyclohexylisocyanate is used as catalyst.

65. A process according to claim 63, wherein 0.001 to 10 mole percent catalyst are used related to the amount of compound Ic.

66. A process according to claim 61, wherein chlorobenzene is used as solvent.

67. A process according to claim 41, wherein the step e) reaction is carried out in the presence of chlorobenzene as solvent.

68. A process according to claim 41, wherein the step e) reaction is carried out at a temperature range from from 20 to 180 °C.

69. A process according to claim 41, wherein the step e) reaction is carried out under normal pressure or an elevated pressure of up to 1 bar.

70. A process according to claim 41, wherein the step e) reaction is carried out in adding the obtained reaction solution with the isocyanate of formula VI to the solution or suspension of the compound of the formula VII.

## Patentansprüche

1. Verfahren zur Herstellung von Verbindungen der Formel Ia
Ar-CHRₐ-CHR_{b}R_{c} (Ia),
worin Rₐ, R_{b} und R_{c} unabhängig voneinander H oder einen bei Hydrierung stabilen Substituenten bedeuten und Ar C₆-C₂₀-Aryl oder C₃-C₂₀-Heteroaryl mit 1 bis 6 Heteroatomen aus der Gruppe O, S und N bedeutet, wobei Aryl und Heteroaryl nicht substituiert sind oder mit Resten substituiert sind, die bei der Hydrierung stabil sind, durch
a) im ersten Schritt Umsetzen von 1 Moläquivalent einer Verbindung der Formel II
Ar-N₂^{⊕} (IIa)
mit mindestens 1 Moläquivalent einer Verbindung der Formel IIIa
CHRₐ=CR_{b}R_{c} (IIIa),
gegebenenfalls in Gegenwart eines inerten Lösungsmittels und in Gegenwart einer katalytischen Menge eines homogenen Palladiumkatalysators und einer Base, ausgewählt aus Alkalimetalisalzen, Erdalkalimetallsalzen und einem tertiären Ammoniumsalz einer Carbonsäure zu einer Verbindung der Formel IVa
Ar-CRₐ=CR_{b}R_{c} (IVa),
und
b) in einem zweiten Schritt Hydrieren der Verbindung der Formel IVa, gegebenenfalls in Gegenwart eines inerten Lösungsmittels und in Gegenwart katalytischer Mengen eines Palladiumhydrierungskatalysators, dadurch gekennzeichnet, daß der homogene Palladiumkatalysator zu unlöslichem Palladiummetall nach der Umsetzung von Schritt a) reduziert wird, das anschließend als heterogener Hydrierungskatalysator verwendet wird.

2. Verfahren nach Anspruch 1, wobei der heterogene Palladiumhydrierungskatalysator in situ aus dem homogenen Palladiumkatalysator in dem Reaktionsgemisch, das von Schritt a) erhalten wird, bei Beginn der Hydrierung durch Einlassen von Wasserstoff gebildet wird.

3. Verfahren nach Anspruch 1, wobei ein Palladiumträgermaterial für den heterogenen Hydrierungskatalysator vor dem Einlassen von Wasserstoff zugegeben wird.

4. Verfahren nach Anspruch 1, wobei Rₐ, R_{b} und R_{c} ausgewählt sind aus H; C₁-C₂₀-Alkyl; C₁-C₂₀-Nitriloalkyl; C₁-C₂₀-Hydroxyalkyl; C₁-C₂₀-Halogenalkyl, Halogen ist vorzugsweise F, Cl oder Br; C₁-C₁₂-Alkyl-COOR_{d}, C₁-C₁₂-Alkyl-CO-NRₑR_{f}, C₁-C₁₂-Alkyl-SO₂OR_{d} oder C₁-C₁₂-Alkyl-SO₂-NRₑR_{f}, wobei R_{d}, Rₑ und R_{f} unabhängig voneinander H, C₁-C₁₂-Alkyl, Phenyl, Benzyl oder Cyclohexyl bedeuten; C₁-C₂₀-Alkyl-CO; C₁-C₂₀-Alkoxy; C₁-C₂₀-Nitriloalkoxy; C₁-C₂₀-Halogenalkoxy, Halogen ist vorzugsweise F, Cl oder Br; C₁-C₂₀-Alkylthio; C₁-C₂₀-Halogenalkylthio, Halogen ist vorzugsweise F, Cl oder Br; -SO₂OR_{d}, -SO₂-NRₑR_{f}, -COOR_{d} oder -CO-NRₑR_{f}, wobei R_{d}, Rₑ und R_{f} die vorstehend genannten Bedeutungen aufweisen; Halogen ist vorzugsweise F, Cl oder Br; -CN; -NRₑR_{f}, wobei Rₑ und R_{f} die vorstehenden Bedeutungen aufweisen; Phenyl oder Benzyl, das unsubstituiert oder mit C₁-C₂₀-Alkyl substituiert ist; C₁-C₂₀-Nitriloalkyl; C₁-C₂₀-Hydroxyalkyl; C₁-C₂₀-Halogenalkyl, Halogen ist vorzugsweise F, Cl oder Br; C₁-C₁₂-Alkyl-COOR_{d}, C₁-C₁₂-Alkyl-CO-NRₑR_{f}, C₁-C₁₂-Alkyl-SO₂OR_{d} oder C₁-C₁₂-Alkyl-SO₂-NRₑR_{f}, wobei R_{d}, Rₑ und R_{f} unabhängig voneinander H, C₁-C₁₂-Alkyl, Phenyl, Benzyl oder Cyclohexyl bedeuten; C₁-C₂₀-Alkyl-CO-; C₁-C₂₀-Alkoxy; C₁-C₂₀-Nitriloalkoxy; C₁-C₂₀-Halogenalkoxy, Halogen ist vorzugsweise F, Cl oder Br; C₁-C₂₀-Alkylthio; C₁-C₂₀-Halogenalkylthio, Halogen ist vorzugsweise F, Cl oder Br; -SO₂OR_{d}, -SO₂-NRₑR_{f}, -COOR_{d} oder -CO-NRₑR_{f}, wobei R_{d}, Rₑ und R_{f} die vorstehend genannten Bedeutungen aufweisen; Halogen ist vorzugsweise F, Cl oder Br; -CN; -NRₑR_{f}, wobei Rₑ und R_{f} die vorstehend genannten Bedeutungen aufweisen, wobei R_{d} ebenfalls -OM oder -O(M₁)_{1/2} wiedergeben kann, wobei M ein Alkalimetallatom oder eine tertiäre Ammoniumgruppe mit 3 bis 18 Kohlenstoffatomen bedeutet und M₁ ein Erdalkalimetallatom ist.

5. Verfahren nach Anspruch 4, wobei die Alkyl-, Alkoxy- und Alkylthiogruppen 1 bis 12 Kohlenstoffatome enthalten.

6. Verfahren nach Anspruch 1, wobei Aryl Ar 6 bis 16 Kohlenstoffatome enthält und Ar monocyclisches oder kondensiertes polycyclisches Aryl bedeutet, wobei das polycyclische Aryl bis zu 3 Ringe enthält.

7. Verfahren nach Anspruch 6, wobei die Ar-Gruppen Naphthyl oder Phenyl sind.

8. Verfahren nach Anspruch 1, wobei Heteroaryl 3 bis 14 Kohlenstoffatome mit 1 bis 4 Heteroatomen aus der Gruppe O, S und N enthält.

9. Verfahren nach Anspruch 1, wobei Heteroaryl monocyclisches oder kondensiertes polycyclisches Heteroaryl ist, wobei das polycyclische Heteroaryl bis zu 3 Ringe enthält.

10. Verfahren nach Anspruch 1 zur Herstellung von Verbindungen der Formel I worin 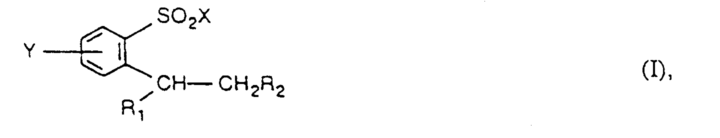
X Hydroxyl, -OM, -O(M₁)_{1/2} oder NH₂ bedeutet, wobei M ein Alkalimetallatom oder eine tertiäre Ammoniumgruppe mit 3 bis 18 Kohlenstoffatomen bedeutet und M₁ ein Erdalkalimetallatom ist,
Y H, Cl, F oder Br bedeutet,
R₁ H, F, Cl, Br oder -COOR₃ darstellt,
R₂ -COO(C₁-C₄-Alkyl), -(CO)R₃ oder C₁-C₂-Alkyl, unsubstituiert oder substituiert mit Halogenatomen bedeutet und
R₃ H oder C₁-C₄-Alkyl darstellt, durch
a) in einem ersten Schritt Umsetzen von 1 Moläquivalent einer Verbindung der Formel II 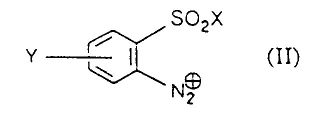 mit mindestens 1 Moläquivalent einer Verbindung der Formel III
CHR₁=CHR₂ (III),
gegebenenfalls in Gegenwart eines inerten Lösungsmittels und in Gegenwart einer katalytischen Menge eines homogenen Palladiumkatalysators und einer Base, ausgewählt aus Alkalimetallsalzen, Erdalkalimetallsalzen und einen tertiären Ammoniumsalz einer Carbonsäure zu einer Verbindung der Formel IV 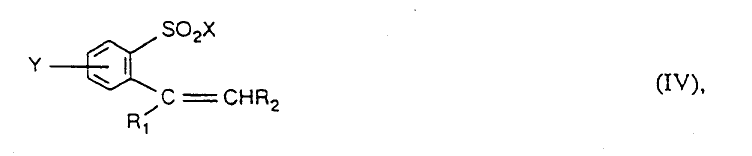 und
b) in einem zweiten Schritt Hydrieren der Verbindung der Formel IV, gegebenenfalls in Gegenwart eines inerten Lösungsmittels und in Gegenwart katalytischer Mengen eines Hydrierungskatalysators, dadurch gekennzeichnet, daß der homogene Palladiumkatalysator zu unlöslichem Palladiummetall in dem Reaktionsgemisch von Schritt a) reduziert wird, das anschließend als heterogener Hydrierungskatalysator eingesetzt wird.

11. Verfahren nach Anspruch 10, wobei der heterogene Palladiumhydrierungskatalysator in situ aus dem homogenen Palladiumkatalysator in dem Reaktionsgemisch, das von Schritt a) erhalten wird, bei Beginn der Hydrierung durch Einlassen von Wasserstoff gebildet wird.

12. Verfahren nach Anspruch 10, wobei M Lithium, Natrium oder Kalium ist.

13. Verfahren nach Anspruch 10, wobei M₁ Magnesium oder Calcium ist.

14. Verfahren nach Anspruch 10, wobei R₁ H ist.

15. Verfahren nach Anspruch 10, wobei R₂ -CF₃, -CF₂Cl, -CFCl₂, -CCl₃, -COO(C₁-C₄-Alkyl) oder -(CO)CH₃ ist.

16. Verfahren nach Anspruch 1, wobei R₁ H ist und R₂ -CF₃ oder -(CO)CH₃ ist.

17. Verfahren nach Anspruch 1, wobei X OH, ONa oder OK ist.

18. Verfahren nach Anspruch 1, wobei Y H ist.

19. Verfahren nach Anspruch 1, wobei der Katalysator in situ oder ex situ durch Reduktion einer Palladium-II-Verbindung und in Gegenwart geeigneter ligandenbildender Verbindung erzeugt wird.

20. Verfahren nach Anspruch 19, wobei die Palladiumverbindungen PdCl₂, PdBr₂, Pd(NO₃)₂, H₂PdCl₄, Pd(OOCCH₃)₂, [PdCl₄]Na₂, [PdCl₄]Li₂, [PdCl₄]K₂, Palladium-II-acetylacetonat, Dichlor-(1,5-cyclooctadien)palladium-II, Dichlorbis-(acetonitril)palladium-II, Dichlorbis-(benzonitril)palladium-II, π-Allylpalladium-II-chloriddimer, Bis-(π-methylallylpalladium-II-chlorid) und π-Allylpalladium-II-acetylacetonat einschließen.

21. Verfahren nach Anspruch 19, wobei die ligandenbildenden Verbindungen ausgewählt sind aus der Gruppe, bestehend aus Olefinen, beschrieben durch die Verbindungen der Formel III, Dibenzylidenaceton (dba), unsubstituiert oder substituiert mit Halogen, C₁-C₄-Alkyl oder C₁-C₄-Alkoxy in den Benzolringen, Phosphiten der Formel P(OR₇), wobei R₇ beispielsweise Phenyl, C₁-C₆-Alkyl oder teil- oder perfluoriertes C₁-C₆-Alkyl bedeutet und CO.

22. Verfahren nach Anspruch 10, wobei die eingesetzten Reduktionsmittel CO, H₂, Formiate, primäre oder sekundäre C₁-C₈-Alkanole, Hydrazin, Amine und Gemische von CO mit Alkanolen oder Wasser sind.

23. Verfahren nach Anspruch 10, wobei der zugegebene Katalysator Pd(dba)₂, Pd(dba)₃-Lösungsmittel, Pd₂(dba)₃ oder Pd₂(dba)₃-Lösungsmittel ist, wobei die Abkürzung "dba" für Dibenzylidenaceton steht, das im aromatischen Teil unsubstituiert ist oder gemäß Anspruch 21 substituiert ist.

24. Verfahren nach Anspruch 10, wobei der Palladiumkatalysator in einer Menge von 0,01 bis 5 Mol-%, bezogen auf das Diazoniumsalz der Formel II, nach Anspruch 10, eingesetzt wird.

25. Verfahren nach Anspruch 10, wobei eine Base vorliegt, die ausgewählt ist aus Li-, Na-, K-, NH₄-, Mg-, Ca- und NH(C₁-C₁₈-Alkyl)₃-Salzen von Carbonsäuren.

26. Verfahren nach Anspruch 25, wobei die Carbonsäure eine C₁-C₄-Carbonsäure oder Benzoesäure ist.

27. Verfahren nach Anspruch 25, wobei Natrium- oder Kaliumacetat als Base vorliegt.

28. Verfahren nach Anspruch 10, wobei ein Palladiumträgermaterial für den heterogenen Hydrierungskatalysator vor Einlassen von Wasserstoff zugegeben wird.

29. Verfahren nach Anspruch 28, wobei Aktivkohle, Ruß, Al₂O₃, SiO₂, Keramik, Glas oder synthetischer oder natürlich vorkommender Zeolith als Katalysatorträgermaterial zugegeben wird.

30. Verfahren nach Anspruch 10, wobei ein kleiner Überschuß an Alken der Formel III verwendet wird.

31. Verfahren nach Anspruch 10, wobei die Reaktionstemperatur für den Kupplungsschritt zwischen -20 und +40°C liegt.

32. Verfahren nach Anspruch 10, wobei die Reaktionstemperatur für den Hydrierungsschritt zwischen Raumtemperatur und 200°C liegt.

33. Verfahren nach Anspruch 10, wobei der Kupplungsschritt bei einem Partialdruck der Kupplungskomponente der Formel III zwischen Atmosphärendruck und 10⁶ Pa ausgeführt wird.

34. Verfahren nach Anspruch 10, wobei die Hydrierungsstufe bei einem Wasserstoffpartialdruck zwischen Atmosphärendruck und 3 x 10⁶ Pa ausgeführt wird.

35. Verfahren nach Anspruch 10, wobei die Reaktion in einem Alkohol als Lösungsmittel ausgeführt wird.

36. Verfahren nach Anspruch 35, wobei das Lösungsmittel Pentanol oder Isopropanol ist.

37. Verfahren nach Anspruch 10, wobei die Reaktion in Butanol, Pentanol, Isopropanol, Acetonitril, Essigsäure oder Dioxan als Lösungsmittel ausgeführt wird.

38. Verfahren nach Anspruch 10, wobei die Reaktion als Eintopfreaktion ausgeführt wird.

39. Verfahren nach Anspruch 10, wobei der homogene Palladiumkatalysator mit Wasserstoff reduziert wird.

40. Verfahren nach Anspruch 10 zur Herstellung von Natrium-2-(3,3,3-trifluorprop-1-yl)-benzolsulfonat.

41. Verfahren zur Herstellung von Verbindungen der Formel V 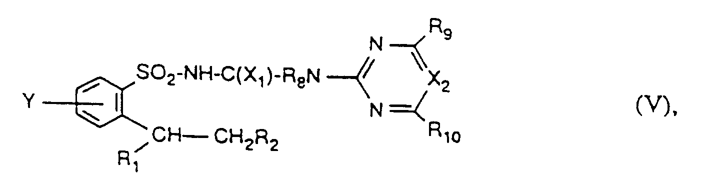 worin
X₁ S oder O bedeutet, X₂ N oder CH bedeutet,
Y H, Cl, F oder Br darstellt,
R₁ H, F, Cl, Br oder -COOR₃ bedeutet,
R₂ -COO(C₁-C₄-Alkyl), -(CO)R₃ oder C₁-C₂-Alkyl, unsubstituiert oder substituiert mit Halogenatomen, bedeutet und
R₃ H oder C₁-C₄-Alkyl darstellt,
R₈ H, C₁-C₃-Alkyl oder C₁-C₃-Alkoxy bedeutet,
R₉ C₁-C₃-Alkyl, C₁-C₃-Halogenalkyl, C₁-C₃-Alkoxy oder C₁-C₃-Halogenalkoxy darstellt und
R₁₀ H, Halogen, NH₂, NH(C₁-C₃-Alkyl), NH(C₁-C₃-Alkyl)₂, C₁-C₃-Alkyl, C₁-C₃-Halogenalkyl, C₁-C₃-Alkoxy oder C₁-C₃-Halogenalkoxy bedeutet, durch
a) in einem ersten Schritt Umsetzen von 1 Moläquivalent einer Verbindung der Formel IIb 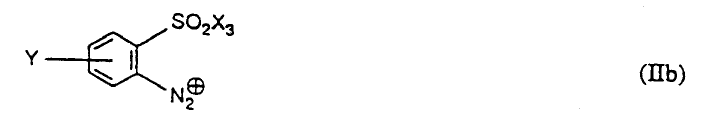 worin X₃ Hydroxyl, -OM oder -O(M₁)_{1/2} wiedergibt, wobei M ein Alkalimetallatom oder eine tertiäre Ammoniumgruppe mit 3 bis 18 Kohlenstoffatomen bedeutet und M₁ ein Erdalkalimetallatom bedeutet, mit mindestens 1 Moläquivalent einer Verbindung der Formel IIIb
CHR₁=CHR₂ (IIIb),
gegebenenfalls in Gegenwart eines inerten Lösungsmittels und in Gegenwart einer katalytischen Menge eines homogenen Palladiumkatalysators und einer Base, ausgewählt aus Alkalimetallsalzen, Erdalkalimetallsalzen und einem tertiären Ammoniumsalz einer Carbonsäure zu einer Verbindung der Formel IVb 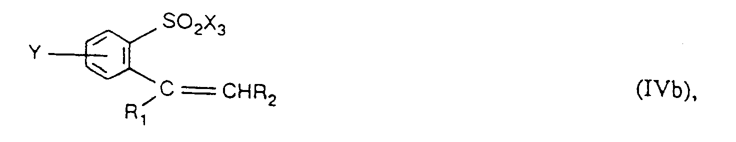 und
b) in einem zweiten Schritt Hydrieren der Verbindung der Formel IVb, gegebenenfalls in Gegenwart eines inerten Lösungsmittels und in Gegenwart katalytischer Mengen eines Hydrierungskatalysators zu einer Verbindung der Formel Ib 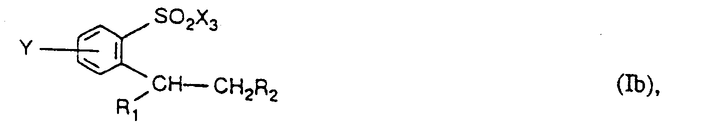
c) in einem dritten Schritt Umsetzen der Verbindung der Formel Ib mit mindestens 1 Mol eines Halogenierungsmittels zu dem Sulfochlorid, das dann mit NH₃ zu dem Sulfonamid der Formel Ic umgesetzt wird 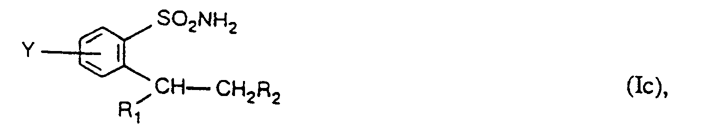
d) Umsetzen der Verbindung der Formel Ic mit COCl₂ oder CSCl₂ zu einer Verbindung der Formel VI 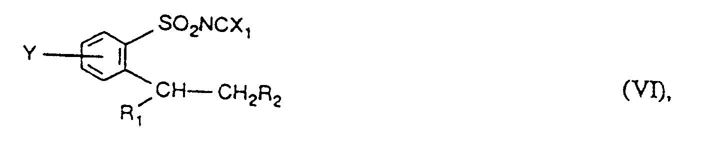 und
e) Umsetzen der Verbindung der Formel VI mit einer Verbindung der Formel VII 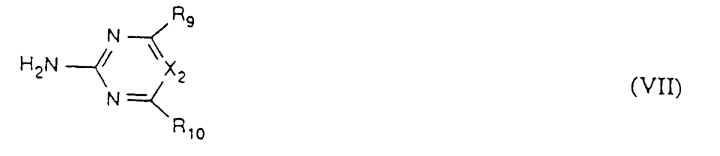 zu der Verbindung der Formel V,
dadurch gekennzeichnet, daß der homogene Palladiumkatalysator zu unlöslichem Palladiummetall in dem Reaktionsgemisch von Schritt a) reduziert wird, das anschließend als heterogener Hydrierungskatalysator eingesetzt wird.

42. Verfahren nach Anspruch 41, dadurch gekennzeichnet, daß der heterogene Palladiumhydrierungskatalysator in der Reaktion von Schritt b) in situ aus dem homogenen Palladiumkatalysator im Reaktionsgemisch, das in Schritt a) erhalten wird, bei Beginn der Hydrierung durch Einlassen von Wasserstoff gebildet wird.

43. Verfahren nach Anspruch 41, wobei ein festes Palladiumträgermaterial für den heterogenen Hydrierungskatalysator vor Beginn der Hydrierung zugegeben wird.

44. Verfahren nach Anspruch 41, wobei X₃ eine Hydroxylgruppe oder eine Gruppe -OM bedeutet, wobei M ein Alkalimetall ist

45. Verfahren nach Anspruch 41, wobei X₃ die Gruppe -ONa bedeutet.

46. Verfahren nach Anspruch 41, wobei X₁ O ist.

47. Verfahren nach Anspruch 41, wobei X₂ N ist.

48. Verfahren nach Anspruch 41, wobei R₈ H ist.

49. Verfahren nach Anspruch 41, wobei R₉ C₁-C₃-Alkyl ist.

50. Verfahren nach Anspruch 49, wobei R₉ Methyl oder Ethyl ist.

51. Verfahren nach Anspruch 41, wobei R₁₀ C₁-C₃-Alkyl oder C₁-C₃-Alkoxy ist.

52. Verfahren nach Anspruch 51, wobei R₁₀ Methyl, Ethyl, Methoxy oder Ethoxy ist.

53. Verfahren nach Anspruch 41 zur Herstellung von N-(4-Methoxy-6-methyl-1,3,5-triazin-2-yl)-N'-[2-(3,3,3-trifluorprop-1-yl)-benzolsulfonyl]-harnstoff.

54. Verfahren nach Anspruch 41, wobei in der Reaktion von Schritt c) das Halogenierungsmittel COCl₂ ist.

55. Verfahren nach Anspruch 54, wobei die Reaktion durch Zugabe von Dimethylformamid katalysiert wird.

56. Verfahren nach Anspruch 55, wobei 0,001 bis 10 Mol-% Dimethylformamid, bezogen auf die Menge von Verbindung Ib, verwendet werden.

57. Verfahren nach Anspruch 54, wobei die Reaktionstemperatur 20 bis 150°C ist.

58. Verfahren nach Anspruch 54, wobei Chlorbenzol als Lösungsmittel verwendet wird.

59. Verfahren nach Anspruch 54, wobei das Sulfochlorid ohne Isolierung in dem erhaltenen Reaktionsgemisch mit wässerigem NH₃ unter Bildung des Sulfonamids der Formel Ic behandelt wird.

60. Verfahren nach Anspruch 59, wobei die Reaktionstemperatur 20 bis 100°C beträgt.

61. Verfahren nach Anspruch 41, wobei die Reaktion in Schritt d) mit einem Überschuß an Phosgen oder Thiophosgen ausgeführt wird.

62. Verfahren nach Anspruch 61, wobei die Reaktionstemperatur 50 bis 180°C beträgt.

63. Verfahren nach Anspruch 61, wobei die Reaktion durch Zugabe von aliphatischen oder cycloaliphatischen Isocyanaten mit 1 bis 10 Kohlenstoffatomen katalysiert wird.

64. Verfahren nach Anspruch 63, wobei Cyclohexylisocyanat als Katalysator verwendet wird.

65. Verfahren nach Anspruch 63, wobei 0,001 bis 10 Mol-% Katalysator, bezogen auf die Menge von Verbindung Ic, verwendet werden.

66. Verfahren nach Anspruch 61, wobei Chlorbenzol als Lösungsmittel verwendet wird.

67. Verfahren nach Anspruch 41, wobei die Reaktion von Schritt e) in Gegenwart von Chlorbenzol als Lösungsmittel ausgeführt wird.

68. Verfahren nach Anspruch 41, wobei die Reaktion in Schritt e) bei einer Temperatur bei 20 bis 180°C ausgeführt wird.

69. Verfahren nach Anspruch 41, wobei die Reaktion in Schritt e) unter Normaldruck oder bei erhöhten Druck bis 1 Bar ausgeführt wird.

70. Verfahren nach Anspruch 41, wobei die Reaktion in Schritt e) bei Zugabe der erhaltenen Reaktionslösung mit dem Isocyanat der Formel VI zu der Lösung oder Suspension der Verbindung der Formel VII ausgeführt wird.

## Revendications

1. Procédé de préparation de composés de formule Ia
Ar-CHRₐ-CHR_{b}R_{c} (Ia),
dans laquelle Rₐ, R_{b} et R_{c} représentent chacun, indépendamment les uns des autres, H ou un substituant stable à l'hydrogénation et Ar représente un groupe aryle en C6-C20 ou hétéroaryle en C3-C20 contenant 1 à 6 hétéroatomes choisis parmi O, S et N, les groupes aryle et hétéroaryle étant non substitués ou portant des substituants stables à l'hydrogénation, dans lequel
a) dans un premier stade, on fait réagir un équivalent molaire d'un composé de formule IIa
Ar-N₂^{⊕} (IIa)
avec au moins un équivalent molaire d'un composé de formule IIIa
CHRₐ=CR_{b}R_{c} (IIIa),
éventuellement en présence d'un solvant inerte, et en présence d'une quantité catalytique d'un catalyseur homogène au palladium et d'une base choisie parmi les sels de métaux alcalins, les sels de métaux alcalino-terreux et les sels d'ammonium tertiaire d'acides carboxyliques, la réaction donnant un composé de formule IVa
Ar-CRₐ=CR_{b}R_{c} (IVa),
et
b) dans un second stade opératoire, on hydrogène le composé de formule IVa, éventuellement en présence d'un solvant inerte, et en présence de quantités catalytiques d'un catalyseur d'hydrogénation au palladium, ce procédé se caractérisant en ce que le catalyseur homogène au palladium est réduit, après la réaction du stade opératoire a), en palladium métallique insoluble qui est ensuite utilisé en tant que catalyseur d'hydrogénation hétérogène.

2. Procédé selon la revendication 1, dans lequel le catalyseur d'hydrogénation hétérogène consistant en palladium est formé in situ à partir du catalyseur homogène au palladium, dans le mélange de réaction obtenu au stade opératoire a), en commençant l'hydrogénation par introduction d'hydrogène.

3. Procédé selon la revendication 1, dans lequel, avant l'introduction d'hydrogène, on ajoute une matière de support pour palladium, prévue pour le catalyseur d'hydrogénation hétérogène.

4. Procédé selon la revendication 1, dans lequel Rₐ, R_{b} et R_{c} sont choisis parmi H; les groupes alkyle en C1-C20; les groupes nitriloalkyle en C1-C20; les groupes hydroxyalkyle en C1-C20; les groupes halogénoalkyle en C1-C20 dans lesquels les halogènes consistent de préférence en F, Cl ou Br; les groupes (alkyle en C1-C12)-COOR_{d}, (alkyle en C1-C12)-CO-NRₑR_{f}, (alkyle en C1-C12)-SO₂OR_{d} ou (alkyle en C1-C12)-SO₂-NRₑR_{f}, dans lesquels R_{d}, Rₑ et R_{f} sont choisis, indépendamment les uns des autres, parmi H, les groupes alkyle en C1-C12, phényle, benzyle ou cyclohexyle ; les groupes (alkyle en C1-C20)-CO; alcoxy en C1-C20; nitriloalcoxy en C1-C20; halogénoalcoxy en C1-C20 dans lesquels les halogènes consistent de préférence en F, Cl ou Br ; les groupes alkylthio en C1-C20; halogénoalkylthio en C1-C20, les halogènes étant de prérérence choisis parmi F, Cl ou Br; les groupes -SO₂OR_{d}, -SO₂NRₑR_{f}, -COOR_{d} ou -CO-NRₑR_{f} dans lesquels R_{d}, Rₑ et R_{f} ont les significations indiquées ci-dessus, les halogènes consistant de préférence en F, Cl ou Br; les groupes -CN; -NRₑR_{f} dans lesquels Rₑ et R_{f} ont les significations indiquées ci-dessus; les groupes phényle ou benzyle non substitués ou substitués par des groupes alkyle en C1-C20; les groupes nitriloalkyle en C1-C20; hydroxyalkyle en C1-C20; halogénoalkyle en C1-C20 dans lesquels les halogènes consistent de préférence en F, Cl ou Br, (alkyle en C1-C12)-COOR_{d}, (alkyle en C1-C12)-CO-NRₑR_{f}, (alkyle en C1-C12)-SO₂OR_{d} ou (alkyle en C1-C12)-SO₂-NRₑR_{f} dans lesquels R_{d}, Rₑ et R_{f} sont choisis, indépendamment les uns des autres, parmi H, les groupes alkyle en C1-C12, phényle, benzyle ou cyclohexyle; les groupes (alkyle en C1-C20)-CO-; les groupes alcoxy en C1-C20; nitriloalcoxy en C1-C20; halogénoalcoxy en C1-C20 dans lesquels les halogènes consistent de préférence en F, Cl ou Br; les groupes alkylthio en C1-C20; halogénoalkylthio en C1-C20 dans lesquels les halogènes consistent de préférence en F, Cl ou Br, les groupes -SO₂OR_{d}, -SO₂-NRₑR_{f}, -COOR_{d} ou -CO-NRₑR_{f} dans lesquels R_{d}, Rₑ et R_{f} ont les significations indiquées ci-dessus; les halogènes consistant de préférence en F, Cl ou Br; les groupes -CN; -NRₑR_{f} dans lesquels Rₑ et R_{f} ont les significations indiquées ci-dessus, R_{d} pouvant également représenter -OM ou -O(M₁)½ dans lesquels M représente un atome de métal alcalin ou un groupe ammonium tertiaire en C3-C18 et M₁ représente un atome de métal alcalino-terreux.

5. Procédé selon la revendication 4, dans lequel les groupes alkyle, alcoxy et alkylthio contiennent de 1 à 12 atomes de carbone.

6. Procédé selon la revendication 1, dans lequel le groupe aryle Ar contient 6 à 16 atomes de carbone et consiste en un groupe aryle monocyclique ou polycyclique condensé, le polycyclique condensé contenant jusqu'à trois cycles.

7. Procédé selon la revendication 6, dans lequel les groupes Ar sont des groupes naphtyle ou phényle.

8. Procédé selon la revendication 1, dans lequel les groupes hétéroaryle contiennent 3 à 14 atomes de carbone et 1 a 4 hétéroatomes choisis parmi O, S et N.

9. Procédé selon la revendication 1, dans lequel le groupe hétéroaryle est monocyclique ou polycyclique condensé le polycyclique condensé contenant jusqu'à trois cycles.

10. Procédé selon la revendication 1 pour la préparation de composés de formule I 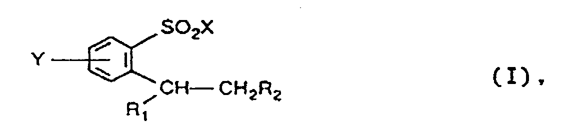 dans laquelle
X représente un groupe hydroxy, -OM, -O(M₁)½ ou NH₂, M représentant un atome de métal alcalin ou un groupe ammonium tertiaire en C3-C18, et M₁ un atome de métal alcalino-terreux,
Y représente H, Cl, F ou Br,
R₁ représente H, F, Cl, Br ou -COOR₃,
R₂ représente -COO-alkyle en C1-C4, -(CO)R₃ ou un groupe alkyle en C1-C2 non substitué ou substitué par des atomes d'halogène et
R₃ représente H ou un groupe alkyle en C1-C4, dans lequel
a) dans un premier stade opératoire, on fait réagir un équivalent molaire d'un composé de formule II 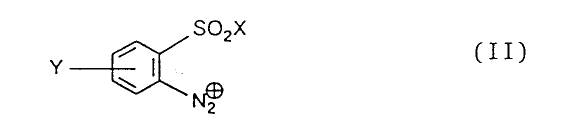 avec au moins un équivalent molaire d'un composé de formule III
CHR₁=CHR₂ (III),
éventuellement en présence d'un solvant inerte, et en présence d'une quantité catalytique d'un catalyseur homogène au palladium et d'une base choisie parmi les sels de métaux alcalins, les sels de métaux alcalino-terreux et les sels d'ammonium tertiaire d'acides carboxyliques, la réaction donnant un composé de formule IV 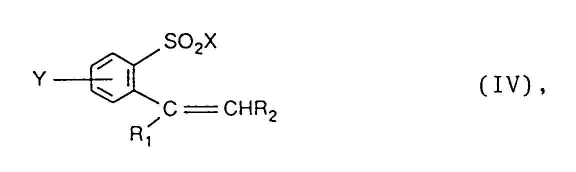 et
b) dans un deuxième stade opératoire, on hydrogène le composé de formule IV, éventuellement en présence d'un solvant inerte et en présence de quantités catalytiques d'un catalyseur d'hydrogénation, ce procédé se caractérisant en ce que le catalyseur homogène au palladium est réduit en palladium métallique insoluble dans le mélange de réaction du stade a) et utilisé ensuite en tant que catalyseur d'hydrogénation hétérogène.

11. Procédé selon la revendication 10, dans lequel le catalyseur hétérogène d'hydrogénation au palladium est formé in situ à partir du catalyseur homogène au palladium dans le mélange de réaction obtenu au stade a) en commençant l'hydrogénation par introduction d'hydrogène.

12. Procédé selon la revendication 10, dans lequel M représente le lithium, le sodium ou le potassium.

13. Procédé selon la revendication 10, dans lequel M₁ représente le magnésium ou le calcium.

14. Procédé selon la revendication 10, dans lequel R₁ représente H.

15. Procédé selon la revendication 10, dans lequel R₂ représente -CH₃, -CF₂Cl, -CFCl₂, -CCl₃, -COO-(alkyle en C1-C4) ou -(CO)CH₃.

16. Procédé selon la revendication 1, dans lequel R₁ représente H et R₂ représente -CF₃ ou -(CO)CH₃.

17. Procédé selon la revendication 1, dans lequel X représente OH, ONa ou OK.

18. Procédé selon la revendication 1, dans lequel Y représente H.

19. Procédé selon la revendication 1, dans lequel le catalyseur est formé in situ ou extérieurement par réduction d'un composé du palladium-II et en présence de composés appropriés à la formation de ligands.

20. Procédé selon la revendication 19, dans lequel les composés du palladium consistent en PdCl₂, PdBr₂, Pd(NO₃)₂, H₂PdCl₄, Pd(OOCCH₃)₂, [PdCl₄]Na₂, [PdCl₄]Li₂, [PdCl₄]K₂, acétylacétonate de palladium-II, dichloro-(1,5-cyclooctadiène)-palladium-II, dichloro-bis-(acétonitrile)palladium-II, dichloro-bis-(benzonitrile)palladium-II, chlorure de π-allylpalladium-II dimère, bis-(chlorure de π-méthylallylpalladium-II) et acétylacétonate de π-allylpalladium-II.

21. Procédé selon la revendication 19, dans lequel les composés formant des ligands sont choisis dans le groupe des oléfines telles que représentées par les composés de formule III, la dibenzylidèneacétone (dba) non substituée ou substituée par des halogènes, des groupes alkyle en C1-C4 ou alcoxy en C1-C4 sur les cycles benzéniques, les phosphites de formule P(OR₇) dans laquelle R₇ représente par exemple un groupe phényle, alkyle en C1-C6 ou alkyle en C1-C6 fluoré en totalité ou en partie, et CO.

22. Procédé selon la revendication 10, dans lequel les agents réducteurs utilisés consistent en CO, H₂, des formiates, des alcanols primaires ou secondaires en C1-C8, l'hydrazine, des amines et des mélanges de CO avec des alcanols ou de l'eau.

23. Procédé selon la revendication 10, dans lequel le catalyseur ajouté consiste en Pd(dba)₂, Pd(dba)₃.solvant, Pd₂(dba)₃ ou Pd₂(dba)₃.solvant, l'abréviation "dba" représentant la dibenzylidèneacétone non substituée ou substituée dans sa partie aromatique comme indiqué dans la revendication 21.

24. Procédé selon la revendication 10, dans lequel le catalyseur au palladium est utilisé en quantité de 0,01 à 5 mol% par rapport au sel de diazonium de formule II selon la revendication 10.

25. Procédé selon la revendication 10, dans lequel on opère en présence d'une base choisie parmi les sels de Li, de Na, de K, de NH₄, de Mg, de Ca et de NH(alkyle en C1-C18)₃ d'acides carboxyliques.

26. Procédé selon la revendication 25, dans lequel l'acide carboxylique est un acide carboxylique en C1-C4 ou l'acide benzoïque.

27. Procédé selon la revendication 25, selon lequel on utilise en tant que base l'acétate de sodium ou de potassium.

28. Procédé selon la revendication 10, dans lequel, avant d'introduire l'hydrogène, on ajoute une matière de support pour palladium prévue pour le catalyseur d'hydrogénation hétérogène.

29. Procédé selon la revendication 28, selon lequel on ajoute, en tant que support du catalyseur, du charbon, du noir de carbone, Al₂O₃, SiO₂, une matière céramique, du verre ou une zéolite synthétique ou naturelle.

30. Procédé selon la revendication 10, selon lequel on utilise un léger excès de l'alcène de formule III.

31. Procédé selon la revendication 10, selon lequel la température observée à la réaction de condensation est comprise entre -20 et +40°C.

32. Procédé selon la revendication 10, dans lequel la température observée à la réaction d'hydrogénation se situe entre la température ambiante et 200°C.

33. Procédé selon la revendication 10, selon lequel l'opération de condensation est effectuée à une pression partielle du composant de couplage de formule III qui se situe entre la pression atmosphérique et 10⁶Pa.

34. Procédé selon la revendication 10, selon lequel l'hydrogénation est effectuée à une pression partielle d'hydrogène comprise entre la pression atmosphérique et 3 x 10⁶Pa.

35. Procédé selon la revendication 10, selon lequel la réaction est effectuée dans un solvant consistant en un alcool.

36. Procédé selon la revendication 35, selon lequel le solvant consiste en pentanol ou isopropanol.

37. Procédé selon la revendication 10, selon lequel la réaction est effectuée dans un solvant qui consiste en butanol, pentanol, isopropanol, acétonitrile, acide éthanoïque ou dioxanne.

38. Procédé selon la revendication 10, dans lequel la réaction est effectuée dans un seul récipient.

39. Procédé selon la revendication 10, selon lequel le catalyseur homogène au palladium est réduit par l'hydrogène.

40. Procédé selon la revendication 10 pour la préparation du 2-(3,3,3-trifluoropropa-1-yl)benzènesulfonate de sodium.

41. Procédé de préparation des composés de formule V 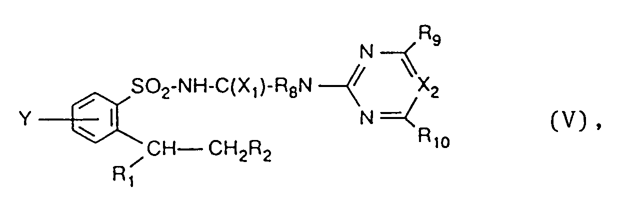 dans laquelle
X₁ représente S ou O, X₂ représente N ou CH,
Y représente H, Cl, F ou Br,
R₁ représente H, F, Cl, Br ou -COOR₃,
R₂ représente -COO-(alkyle en C1-C4), -(CO)R₃ ou un groupe alkyle en C1-C2 non substitué ou substitué par des atomes d'halogène et
R₃ représente H ou un groupe alkyle en C1-C4,
R₈ représente H, un groupe alkyle en C1-C3 ou alcoxy en C1-C3,
R₉ représente un groupe alkyle en C1-C3, halogénoalkyle en C1-C3, alcoxy en C1-C3 ou halogénoalcoxy en C1-C3 et
R₁₀ représente H, un halogène, NH₂, NH(alkyle en C1-C3), NH(alkyle en C1-C3)₂, alkyle en C1-C3, halogénoalkyle en C1-C3, alcoxy en C1-C3 ou halogénoalcoxy en C1-C3, selon lequel
a) dans un premier stade opératoire, on fait réagir un équivalent molaire d'un composé de formule IIb 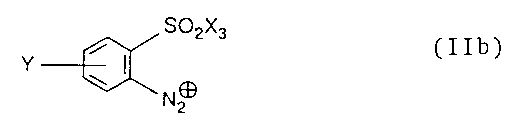 dans laquelle X₃ représente un groupe hydroxy, -OM ou -O(M₁)½, M représentant un atome de métal alcalin ou un groupe ammonium tertiaire en C3-C18 et M₁ un atome de métal alcalino-terreux, avec au moins un équivalent molaire d'un composé de formule IIIb
CHR₁=CHR₂ (IIIb),
éventuellement en présence d'un solvant inerte, et en présence d'une quantité catalytique d'un catalyseur homogène au palladium et d'une base choisie parmi les sels de métaux alcalins, les sels de métaux alcalino-terreux et les sels d'ammonium tertiaire d'acides carboxyliques, la réaction donnant un composé de formule IVb 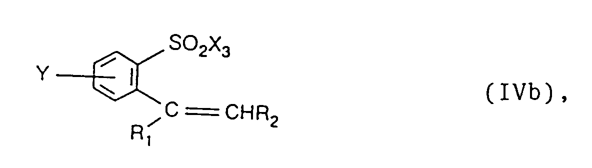 et
b) dans un second stade opératoire, on hydrogène le composé de formule IVb, éventuellement en présence d'un solvant inerte, et en présence de quantités catalytiques d'un catalyseur d'hydrogénation, la réaction donnant un composé de formule Ib 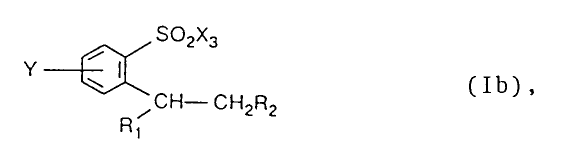
c) dans un troisième stade opératoire, on fait réagir le composé de formule Ib avec au moins une mole d'un agent halogénant, ce qui donne le sulfochlorure correspondant qu'on fait ensuite réagir avec NH₃, ce qui donne le sulfonamide de formule Ic 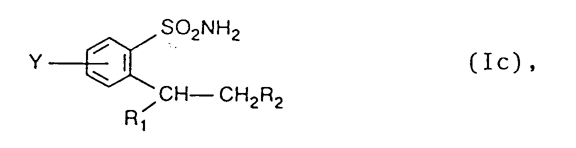
d) on fait réagir le composé de formule I_{c} avec COCl₂, la réaction donnant un composé de formule VI 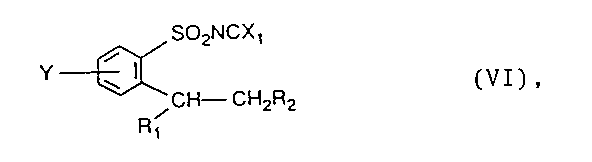 et
e) on fait réagir le composé de formule VI avec un composé de formule VII 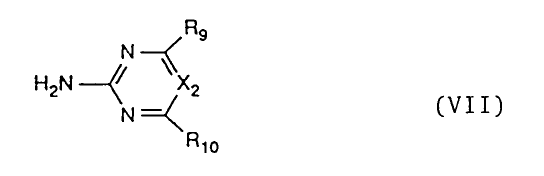 ce qui donne le composé de formule V,
ce procédé se caractérisant en ce que le catalyseur homogène au palladium est réduit en palladium métallique insoluble dans le mélange de réaction du stade a) et utilisé ensuite en tant que catalyseur d'hydrogénation hétérogène.

42. Procédé selon la revendication 41, caractérisé en ce que le catalyseur d'hydrogénation hétérogène au palladium de la réaction du stade b) est formé in situ à partir du catalyseur homogène au palladium dans le mélange de réaction obtenu au stade a) en commençant l'hydrogénation par introduction d'hydrogène.

43. Procédé selon la revendication 41, dans lequel, avant de commencer à hydrogéner, on ajoute une matière de support solide pour palladium prévue pour le catalyseur d'hydrogénation hétérogène.

44. Procédé selon la revendication 41, dans lequel X₃ représente un groupe hydroxy ou un groupe -OM dans lequel M est un métal alcalin.

45. Procédé selon la revendication 41, dans lequel X₃ représente le groupe -ONa.

46. Procédé selon la revendication 41, dans lequel X₁ représente O.

47. Procédé selon la revendication 41, dans lequel X₂ représente N.

48. Procédé selon la revendication 41, dans lequel R₈ représente H.

49. Procédé selon la revendication 41, dans lequel R₉ représente un groupe alkyle en C1-C3.

50. Procédé selon la revendication 49, dans lequel R₉ représente un groupe méthyle ou éthyle.

51. Procédé selon la revendication 41, dans lequel R₁₀ représente un groupe alkyle en C1-C3 ou alcoxy en C1-C3.

52. Procédé selon la revendication 51, dans lequel R₁₀ représente un groupe méthyle, éthyle, méthoxy ou éthoxy.

53. Procédé selon la revendication 41, pour la préparation de la N-(4-méthoxy-6-méthyl-1,3,5-triazine-2-yl)-N'-[2-(3,3,3-trifluoropropa-1-yl)-benzènesulfonyl]-urée.

54. Procédé selon la revendication 41, dans lequel, à la réaction du stade opératoire c), l'agent halogénant est COCl₂.

55. Procédé selon la revendication 54, selon lequel la réaction est catalysée par addition de diméthylformamide.

56. Procédé selon la revendication 55, dans lequel on utilise de 0,001 à 10 mol% de diméthylformamide par rapport à la quantité du composé Ib.

57. Procédé selon la revendication 54, dans lequel la température de réaction va de 20 à 150°C.

58. Procédé selon la revendication 54, dans lequel on utilise en tant que solvant du chlorobenzène.

59. Procédé selon la revendication 54, dans lequel le sulfochlorure est traité sans être isolé, dans le mélange de réaction obtenu, par l'ammoniaque pour formation de sulfonamide de formule Ic.

60. Procédé selon la revendication 59, dans lequel la température de réaction va de 20 à 100°C.

61. Procédé selon la revendication 41, dans lequel la réaction du stade opératoire d) est réalisée avec un excès de phosgène ou de thiophosgène.

62. Procédé selon la revendication 61, dans lequel la température de réaction va de 50 à 180°C.

63. Procédé selon la revendication 61, dans lequel la réaction est catalysée par addition d'isocyanates aliphatiques ou cycloaliphatiques en C1-C10.

64. Procédé selon la revendication 63, dans lequel le catalyseur utilisé est l'isocyanate de cyclohexyle.

65. Procédé selon la revendication 63, dans lequel on utilise le catalyseur en quantité de 0,001 à 10 mol% par rapport à la quantité du composé Ic.

66. Procédé selon la revendication 61, dans lequel le solvant utilisé est le chlorobenzène.

67. Procédé selon la revendication 41, selon lequel la réaction du stade opératoire e) est effectuée en présence de chlorobenzène qui sert de solvant.

68. Procédé selon la revendication 41, dans lequel la réaction du stade opératoire e) est réalisée dans un intervalle de température de 20 à 180°C.

69. Procédé selon la revendication 41, dans lequel la réaction du stade opératoire e) est effectuée à pression normale ou sous une pression manométrique allant jusqu'à 1 bar.

70. Procédé selon la revendication 41, dans lequel la réaction du stade opératoire e) est réalisée par addition de la solution obtenue à la réaction de formation de l'isocyanate de formule VI à la solution ou suspension du composé de formule VII.
